(19) Europäisches Patentamt
European Patent Office
Office européen des brevets

(11) EP 1 955 691 A1

(12) **EUROPEAN PATENT APPLICATION**
published in accordance with Art. 158(3) EPC

(43) Date of publication:
**13.08.2008 Bulletin 2008/33**

(21) Application number: **06822845.1**

(22) Date of filing: **26.10.2006**

(51) Int Cl.:
***A61K 8/37** (2006.01)* ***A61K 8/06** (2006.01)*
***A61Q 17/04** (2006.01)*

(86) International application number:
**PCT/JP2006/321925**

(87) International publication number:
**WO 2007/060823 (31.05.2007 Gazette 2007/22)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.11.2005 JP 2005338363**
**24.11.2005 JP 2005338364**

(71) Applicant: **Shiseido Company, Limited**
**Chuo-ku**
**Tokyo 104-8010 (JP)**

(72) Inventors:
- **OMURA, Takayuki,**
**c/o SHISEIDO RESEARCH CENTER**
**Yokohama-shi**
**Kanagawa 224-8558 (JP)**
- **TAKAKURA, Yoshihito,**
**c/o SHISEIDO RESEARCH CENTER**
**Yokohama-shi**
**Kanagawa 224-8558 (JP)**

(74) Representative: **Caen, Thierry Alain et al**
**Cabinet Santarelli**
**14, avenue de la Grande Armée**
**75017 Paris (FR)**

# (54) EXTERNAL PREPARATION FOR SKIN

(57) An external preparation for the skin containing isononyl 2-ethylhexanoate and/or 2-ethylhexyl 2-ethylhexanoate (i.e., component (a)); an oil-in-water emulsion type skin cosmetic composition containing the ingredient (a), (b) one or more ingredients selected from higher fatty acids and higher alcohols which are solid to semi-solid at an ordinary temperature (25°C), (c) a homopolymer, copolymer, cross polymer, or mixture containing one or more ingredients selected from 2-acrylamide-2-methylpropane sulfonic acid and acrylic acid and its derivatives as constituent units and (d) one or more ingredients selected from nonionic surfactants having an HLB value of 9 or more; and an oil-in-water or a water-in-oil emulsion type sunscreen cosmetic composition containing (a) 2-ethylhexyl 2-ethylhexanoate and/or isononyl 2-ethylhexanoate, (b) an ultraviolet absorber, (c) an ultraviolet scatterer and (d) a silicone oil.

EP 1 955 691 A1

## Description

TECHNICAL FIELD

The present invention relates to an external preparation for the skin and an oil-in-water emulsion type skin cosmetic composition containing a specific 2-ethylhexanoate ester. Further, the present invention relates to an oil-in-water or water-in-oil emulsion type sunscreen cosmetic composition, more particularly the present invention relates to an oil-in-water or water-in-oil emulsion type sunscreen cosmetic composition which is superior in useability and formulation stability and can realize a high SPF value.

BACKGROUND ART

To create a moist body, smooth slip, emollient sensation and the like in oil-in-water emulsion type skin cosmetic composition of the past, the technique has been adopted of emulsifying stearic acid, palmitic acid, myristic acid, behenic acid and other higher fatty acids, petrolatum, Carnauba wax, candelilla wax, ceresin, microcrystalline wax and other waxes, lauryl alcohol, myristyl alcohol, palmityl alcohol, stearyl alcohol, behenyl alcohol and other higher alcohols and other solid oils by an emulsifier. Further, in order to suppress crystal precipitation of these solid oils along with time, liquid paraffin, squalane or another hydrocarbon oil liquid at ordinary temperature and having a good compatibility with solid oils or cetyl palmitate, isopropyl isostearate, isodecyl pivalate, oleyl oleate or another ester oil with a relatively long carbon chain where the carbon chain is of the same extent as solid oils is being blended in to attempt to avoid crystal precipitation of solid oils (for example, see "Current Cosmetic Science" (Revised and Enlarged Edition II), The Society of Cosmetic Chemists of Japan ed., published by Yakuji Nippo Limited, July 10, 1992, p. 49).

However, while an oil-in-water emulsion type skin cosmetic composition prepared by a method such as the above has superior stability and an emollient sensation when applied to the skin, it is poor in slip and affinity with the skin, has stickiness, and is not satisfactory in the point of useability. On the other hand, when any solid oil is not formulated, the cosmetic composition is superior in the points of being good in shelf life, slip and affinity with the skin and being free of stickiness, but has the problem that it has no emollient sensation.

The ultraviolet rays reaching the surface of the earth includes UV-A (320 to 400 nm) and UV-B (290 to 320 nm). Among these, if excessively exposed, UV-A causes erythema at the skin, causes an acute inflammatory response, then causes darkening and is known to be one of the causes of skin cancer. Further, it is clear that UV-B only slightly causes erythema, but oxidizes the reduced melanin in the skin to cause deposition of melanin pigment and darkening and that prolonged exposure is a cause of premature skin aging. In this way, as the effects that ultraviolet rays have on the skin have become clearer, the demand for sunscreen cosmetic compositions having a high ultraviolet ray blocking effect has been increasing. As an indicator of protection of the skin from ultraviolet rays, generally the SPF (Sun Protection Factor) value is used. The higher the SPF value, the higher the sunscreen effect.

Normally sunscreen cosmetic compositions includes ultraviolet absorbers and ultraviolet scatterers to block ultraviolet rays from striking the skin and obtain a high SPF value. Ultraviolet absorbers block ultraviolet rays by absorbing light energy, but, generally ultraviolet absorbers have a highly polar absorption band, have skin irritability, and have other unpreferable aspects in terms of safety to the skin. Because of this, silicone oil etc. free of skin irritation and having good useability are being used in combination. However, if a high polarity oil containing an ultraviolet absorber and a nonpolar oil containing a silicone oil are used in combination, the cosmetic composition becomes inferior in the uniformity of the oil phase, problems arise in the emulsion stability and the problem arises of uneven coating on the skin.

On the other hand, ultraviolet scatterers block ultraviolet rays by dispersing the ultraviolet rays. In the past, zinc oxide, titanium dioxide, kaolin, calcium carbonate and other inorganic pigments have been used. These can block ultraviolent rays over a broad spectrum. Furthermore, these are inert, so are high in skin safety and are useful ultraviolet blocking agents.

However, these inorganic pigments, particularly titanium dioxide and zinc oxide, are high in ultraviolet blocking effect as mentioned above, but are also large in covering power (concealing power), and therfore, if applied to the skin, the cosmetic film whitens. This is a cause of a grainy finish. Because of this, it has been proposed to use titanium dioxide or zinc oxide made into fine particles (e.g., see Japanese Patent Publication (B2) No. 47-42502, Japanese Patent Publication (A) No. 49-450 and Japanese Patent Publication (A) No. 64-7941). It is known that the finer these particles, the greater the blocking effect of ultraviolet rays and the higher the light transmission at the visible light region, the higher the transparency.

However, these fine particles of inorganic pigments generally are high in cohesiveness and are difficult to stably disperse in cosmetic compositions and other blends in the fine particle state. Therefore, at present, the ultraviolet blocking effect and transparency are not been obtained as expected. Further, these fine particle pigments are high in refractive index of light, and therefore, when used in a large amount, the concealing power becomes high, a grainy finish etc. is caused, and an unnatural cosmetic finish results. Furthermore, the agglomeration of the particles impedes the spreadability of the cosmetic composition, causes a scratching feeling when applied to the skin, and causes other problems in useability.

Because of this, these ultraviolet scatterers are inherently restricted in amounts formulated. It is not possible to provide a sunscreen cosmetic composition which sufficiently realizes the desired ultraviolet blocking effect. In order to solve this problem, as technology for obtaining a sunscreen cosmetic having a superior oil phase dispersibility and useability, it has been proposed to use a nonanoate ester (e.g., see Japanese Patent Publication (A) No. 2000-169353). However, even by this method, the aqueous phase and oil phase uniformity and in turn the emulsion stability and the dispersibility of zinc oxide or titanium dioxide cannot be said to be sufficient.

DISCLOSURE OF THE INVENTION

The present invention was made in consideration of the above situation and the object thereof is to provide an oil-in-water emulsion type skin cosmetic composition superior in shelf life and useability (slip, affinity with the skin, lack of stickiness, clean feeling, emollient sensation, and firm feeling). Another, object of the present invention is to provide an external preparation for the skin since the inventors, as explained later, discovered external preparations for the skin extremely superior, in particular, in affinity with the skin during research and development for application to the above oil-in-water type emulsion skin cosmetics.

The object of the present invention is to provide a sunscreen cosmetic composition sufficiently exhibiting the superior ultraviolet blocking effect of an ultraviolet absorber and ultraviolet scatterer, good affinity with the skin, and free from a stickiness, that is, superior in useability.

In more detail, it provides an emulsified composition stable even in a system combining a high polarity oil containing an ultraviolet absorbent and a nonpolar oil containing a silicone oil as an agent for improving useability and further provides a cosmetic composition, in which an ultraviolet scatterer such as titanium dioxide or zinc oxide is stabily and uniformly dispersed even if formulated, in which redispersibility is improved so as to realize a high SPF value with a natural finish free of graininess and having transparency, in which affinity with the skin and slip are good, and in which a clean feeling of use free of stickiness is realized.

The present inventors engaged in intensive research and, as a result, discovered that a specific 2-ethylhexanoate ester has the superior effects of being superior in affinity with the skin, superior in compatibility of polar oil/nonpolar oils and superior in improvement of powder dispersion, whereby the present invention is completed.

Namely, the present invention provides an external preparation for the skin comprising isononyl 2-ethylhexanoate and/or 2-ethylhexyl 2-ethylhexanoate.

Further, the present invention provides the above external preparation for the skin comprising isononyl 2-ethylhexanoate and/or 2-ethylhexyl 2-ethylhexanoate in an amount of 1.0 to 20.0 mass%.

Further, the present invention provides an oil-in-water emulsion type skin cosmetic composition comprising (a) isononyl 2-ethylhexanoate and/or 2-ethylhexyl 2-ethylhexanoate, (b) one or more ingredients selected from higher fatty acids and higher alcohols which are solid to semi-solid at an ordinary temperature (25°C), (c) a homopolymer, copolymer, cross polymer or mixture containing one or more ingredients selected from 2-acrylamide-2-methylpropane sulfonic acid (hereinbelow referred to as "AMPS") and acrylic acid and its derivatives as constituent units, and (d) one or more ingredients selected from nonionic surfactants having an HLB value of 9 or more.

Further, the present invention provides the above oil-in-water emulsion type skin cosmetic composition containing the ingredient (a) in an amount of 1.0 to 20.0 mass%, the ingredient (b) in an amount of 0.5 to 8.0 mass%, the ingredient (c) in an amount of 0.1 to 5.0 mass% and the ingredient (d) in an amount of 0.5 to 8.0 mass% in the total mass of the cosmetic.

Further, the present invention provides the above oil-in-water emulsion type skin cosmetic composition, wherein the ingredient (c) is one or more ingredients selected from a vinyl pyrrolidone/AMPS copolymer, dimethylacrylamide/AMPS copolymer, acrylate amide/AMPS copolymer, cross polymer of dimethylacrylamide/AMPS cross-linked by methylene bisacrylamide, a mixture of polyacrylate amide and sodium polyacrylate, sodium acrylate/AMPS copolymer, hydroxyethyl acrylate/AMPS copolymer, ammonium polyacrylate, polyacrylamide/ammonium acrylate copolymer and acrylamide/sodium acrylate copolymer.

Further, the present invention provides the above oil-in-water emulsion type skin cosmetic composition wherein the ingredient (d) is one or more ingredients selected from polyoxyethylene adducts and polyethyleneglycol adducts.

Further, the present invention provides the above oil-in-water emulsion type skin cosmetic composition which uses as the ingredient (d) a combination of a nonionic surfactant having an HLB value of 9 to less than 15 and a nonionic surfactant having an HLB value of 15 or more.

The present inventors engaged in intensive research, in order to solve the above problems, and discovered that by using 2-ethylhexyl 2-ethylhexanoate and/or isononyl 2-ethylhexanoate to prepare a sunscreen cosmetic composition of an oil-in-water type or a water-in-oil type, the compatibility between a high polarity oil containing an ultraviolet absorber and a nonpolar oil containing a silicone oil of a useability improving agent is good, the emulsion stability is

superior, and the dispersibility of titanium dioxide and zinc oxide is superior. In particular, the inventors discovered that a combination is obtained having a superior dispersability in regards to even generally strongly cohesive fine particulate matter, good in stability and in affinity with the skin and slip as a sunscreen cosmetic composition in the presence of a silicone-based surfactant, and having a clean feel and no stickiness, that is, superior in the two aspects of useability. The present invention was developed based on such knowledge.

Namely, the present invention provides an oil-in-water or a water-in-oil emulsion type sunscreen cosmetic composition containing (a) 2-ethylhexyl 2-ethylhexanoate and/or isononyl 2-ethylhexanoate, (b) an ultraviolet absorber, (c) an ultraviolet scatterer and (d) a silicone oil.

Further, the present invention provides the above sunscreen cosmetic composition, wherein the ingredient (b) is one or more ingredients selected from para-aminobenzoic acid derivatives, salicylic acid derivatives, cinnamic acid derivatives, β,β-diphenyl acrylate derivatives, benzophenone derivatives, benzylidene camphor derivatives, phenylbenzimidazole derivatives, triazine derivatives, phenylbenzotriazole derivatives, anthranil derivatives, imidazoline derivatives, benzal malonate derivatives and 4,4-diarylbutadiene derivatives.

Further, the present invention provides the above sunscreen cosmetic composition, wherein the ingredient (c) is zinc oxide and/or titanium dioxide.

Further, the present invention provides the above sunscreen cosmetic composition, wherein the zinc oxide has an average primary particle size of 5 to 40 nm.

Further, the present invention provides the above sunscreen cosmetic composition, wherein the titanium dioxide has an average primary particle size of 5 to 30 nm.

Further, the present invention provides the above sunscreen cosmetic composition containing the ingredient (a) in an amount of 1 to 60 mass%, the ingredient (b) in an amount of 3 to 20 mass%, the ingredient (c) in an amount of 0.5 to 50 mass% and the ingredient (d) in an amount of 1 to 70 mass%, in the total mass of the cosmetic composition.

Further, the present invention provides the above sunscreen cosmetic composition further containing (e) one or more ingredients selected from a silicone-based surfactant, glycerin or a polyglyceryl fatty acid ester.

Further, the present invention provides the above sunscreen cosmetic composition, wherein the silicone-based surfactant as the ingredient (e) is one or more ingredients selected from poly(oxyethylene-oxypropylene) methyl polysiloxane copolymers, polyoxyethylene methyl polysiloxane copolymers, silicone chain branched type methyl polysiloxane copolymers, alkyl chain-silicone chain branched type polyoxyethylene methyl polysiloxane copolymers, cross-linkable polyoxyethylene methyl polysiloxane, cross-linked type polyoxyethylene methyl polysiloxane containing alkyl groups, branched type polyglycerin modified silicone, cross-linkable polyglycerin modified silicone, cross-linkable polyglycerin modified silicone containing alkyl groups and alkyl group branched type polyglycerin modified silicone.

Further, the present invention provides the above sunscreen cosmetic composition containing the ingredient (e) in an amount of 0.01 to 20 mass% in the total mass of the cosmetic.

According to the present invention, there is provided an external preparation for the skin particularly superior in affinity with the skin. Further, according to the present invention, there is provided an oil-in-water emulsion type skin cosmetic composition having superior shelf life and useability (slip on skin, affinity with the skin, lack of stickiness, clean feeling, emollient sensation and firm feeling).

According to the present invention, there is provided a sunscreen cosmetic composition sufficiently exhibiting the superior ultraviolet blocking effect of an ultraviolet absorbent and ultraviolet scatterer, good in affinity with the skin and free of stickiness, that is, superior in useability, and furthermore superior in dispersibility and stability.

## BEST MODE FOR CARRYING OUT THE INVENTION

The present invention will now be explained in detail.

### External Preparation for Skin

The external preparation for the skin of the present invention can use one or both of isononyl 2-ethylhexanoate and 2-ethylhexyl 2-ethylhexanoate.

Isononyl 2-ethylhexanoate is a compound having the following Formula (I):

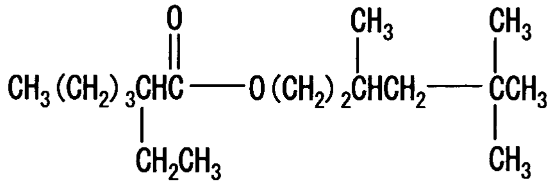

(I)

Further, 2-ethylhexyl 2-ethylhexanoate is a compound having the following Formula (II):

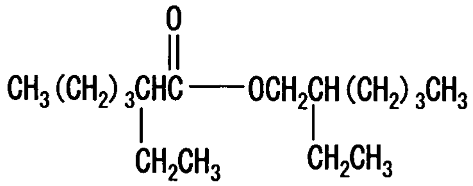

(II)

Conventionally, to improve affinity with the skin and other aspects of useability, examples for formulating esters of isononanoic acid having a carbon chain length of 9 or other low molecular esters in the external preparation for the skin were often observed.

The present invention selects and uses, in particular, the esters having the above formulae (I) and (II) from the esters of 2-ethylhexanoic acid (i.e., carbon chain length=8) having shorter carbon chain lengths than the above conventionally used esters of isononanoic acid. These two esters have respective molecular weights of 270 and 256 and are included in the smallest class of molecular weight in the conventional ester oils which can be used in cosmetic compositions from the viewpoint of safety. Even if compared with the nonanoate esters which have conventionally been widely used in external preparations for the skin, they are smaller in molecular weight. To that extent, they have, in terms of useability, a clean feeling and freshness, and no stickiness and furthermore have superior effects in terms of affinity with the skin, compatibility of polar oil/nonpolar oil, and improvement of powder dispersion.

In particular, the isononyl 2-ethylhexanoate having the above formula (I) has not been listed in any official compendiums. There has never been any external preparation for the skin which contains that compound.

The formulation amount of isononyl 2-ethylhexanoate and 2-ethylhexyl 2-ethylhexanoate is preferably 1.0 to 20.0 mass% in the external preparation for the skin, more preferably 3.0 to 15.0 mass%. If the amount is less than 1.0 mass%, it is difficult to sufficiently realize the effect on affinity with the skin, while if more than 20.0 mass%, the stability of the system sometimes becomes poor.

Oil-in-Water Emulsion Type Skin Cosmetic Composition

The oil-in-water emulsion type skin cosmetic composition of the present invention contains (a) isononyl 2-ethylhexanoate and/or 2-ethylhexyl 2-ethylhexanoate, (b) one or more ingredients selected from higher fatty acids and higher alcohols solid to semi-solid at ordinary temperature (25°C), (c) a homopolymer, copolymer, cross polymer or mixture containing one or more ingredients selected from 2-acrylamide-2-methylpropane sulfonic acid (i.e., AMPS) and acrylic acid and its derivatives as a constituent unit, and (d) one or more ingredients selected from nonionic surfactants having an HLB value of 9 or more.

The ingredient (a) is as explained above, as an ingredient of the external preparation for the skin. The formulation amount of the ingredient (a) is preferably 1.0 to 20.0 mass%, more preferably 3.0 to 15.0 mass%, based upon the total mass of the cosmetic composition. If less than 1.0 mass%, it is difficult for the effect on affinity with the skin to be sufficiently realized, while if more than 20.0 mass%, the stability of the system sometimes becomes poor.

The ingredient (b) is a higher fatty acid or higher alcohol solid to semi-solid at ordinary temperature (25°C) .

As higher fatty acids, caprylic acid, capric acid, lauric acid, myristic acid, palmitic acid, stearic acid, margaric acid, arachidic acid, behenic acid, lignoceric acid, cerotic acid, montanic acid, melissic acid and the like may be mentioned, but the invention is not limited to these examples.

As higher alcohols, lauryl alcohol, myristyl alcohol, cetyl alcohol, stearyl alcohol, behenyl alcohol, arachyl alcohol, batyl alcohol, chimyl alcohol, carnaubyl alcohol, ceryl alcohol, corianyl alcohol, myricyl alcohol, lacceryl alcohol, elaidyl alcohol, isostearyl glyceryl ether, octyl alcohol, triacontyl alcohol, selachyl alcohol, cetostearyl alcohol, oleyl alcohol, lanolin alcohol, hydrogenated lanolin alcohol, hexyl decanol, octyl decanol and the like can be illustrated, but

the invention is not limited to these examples. The ingredient (b) is one or a blend of two or more of these.

The amount of the (b) ingredient blended is preferably 0.5 to 8.0 mass%, more preferably 1.0 to 5.0 mass%, based upon the total mass of the cosmetic composition. If less than 0.5 mass%, the result is inferior in solidification action of the internal oil phase, a problem arises in stability, and, in terms of useability as well, it is difficult to provide the emollient sensation to the skin, while even if formulated in more than 8.0 mass%, not only is no effect commensurate to the increase of the amount (solidification action of internal oil phase, useability, etc.) observed, but also crystal precipitation and other aspects of stability tend to become poor.

The ingredient (c) is a homopolymer, copolymer, cross polymer or mixture containing one or more ingredients selected from 2-acrylamide-2-methylpropane sulfonic acid (i.e., AMPS) and acrylic acid and its derivatives as a constituent unit. As specific examples of the ingredient (c), a vinyl pyrrolidone/AMPS copolymer, dimethylacrylamide/AMPS copolymer, acrylate amide/AMPS copolymer, cross polymer of dimethylacrylamide/AMPS cross-linked by methylene bisacrylamide, a mixture of polyacrylate amide and sodium polyacrylate, sodium acrylate/AMPS copolymer, hydroxyethyl acrylate/AMPS copolymer, ammonium polyacrylate, polyacrylamide/ammonium acrylate copolymer, acrylamide/sodium acrylate copolymer and the like may be mentioned. Among these, the homopolymer of AMPS, vinyl pyrrolidone/AMPS copolymer, dimethylacrylamide/AMPS copolymer, sodium acrylate/AMPS copolymer, cross polymer of dimethylacrylamide/AMPS cross-linked by methylene bisacrylamide, etc. are preferable. The ingredient (c) is one or a blend of two or more of these.

The formulation amount of the ingredient (c) is preferably 0.1 to 5.0 mass%, more preferably 0.3 to 3.0 mass%, based upon the total mass of the cosmetic composition. If less than 0.1 mass%, phenomena such as separation, graininess, emulsion breakdown and agglomeration easily occur, which is not desireable from the viewpoint of stability, while even if formulated over 5.0 mass%, the effect of the present invention is not strengthened, but conversely a stickiness is felt.

The nonionic surfactant having an HLB value of 9 or more of the (d) ingredient is not particularly limited so long as it can be generally formulated into a cosmetic composition. For example, the following can be mentioned. However, the invention is not limited to these Examples. Note that HLB is calculated by the Kawakami formula expressed by the following Equation 1:

$$HLB = 7+11.7\cdot\log\ (MW/MO)$$

wherein, MW represents the molecular weight of the hydrophilic region and MO represents the molecular weight of the lipophilic region.

Hexaglyceryl monolaurate (HLB value 14.5), hexaglyceryl monomyristate (HLB value 11), hexaglyceryl monostearate (HLB value 9.0), hexaglyceryl monooleate (HLB value 9.0), decaglyceryl monolaurate (HLB value 15.5), decaglyceryl monomyristate (HLB value 14.0), decaglyceryl monostearate (HLB value 12.0), decaglyceryl monoisostearate (HLB value 12.0), decaglyceryl monooleate (HLB value 12.0), decaglyceryl distearate (HLB value 9.5), decaglyceryl disisotearate (HLB value 10.0) and other polyglyceryl fatty acid esters.

Polyoxyethylene 5 mol added (expressed as "POE (5)", the same herein below) glyceryl monostearate (HLB value 9.5), POE (15) glyceryl monostearate (HLB value 13.5), POE (5) glyceryl monooleate (HLB value 9.5), POE (15) glyceryl monooleate (HLB value 14.5) and other polyoxyethylene glyceryl fatty acid esters.

POE (20) sorbitan cocoate (HLB value 16.9), POE (20) sorbitan monopalmitate (HLB value 15.6), POE (20) sorbitan monostearate (HLB value 14.9), POE (6) sorbitan monostearate (HLB value 9.5), POE (20) sorbitan tristearate (HLB value 10.5), POE (20) sorbitan monoisostearate (HLB value 15.0), POE (20) sorbitan monooleate (HLB value 15.0), POE (6) sorbitan monooleate (HLB value 10.0), POE (20) sorbitan trioleate (HLB value 11.0) and other polyoxyethylene sorbitan fatty acid esters.

POE (6) sorbitol monolaurate (HLB value 15.5), POE (60) sorbital tetrastearate (HLB value 13.0), POE (30) sorbitol tetraoleate (HLB value 11.5), POE (40) sorbitol tetraoleate (HLB value 12.5), POE (60) sorbitol tetraoleate (HLB value 14.0) and other polyoxyethylene sorbitol fatty acid esters.

POE (10) lanolin (HLB value 12.0), POE (20) lanolin (HLB value 13.0), POE (30) lanolin (HLB value 15.0), POE (5) lanolin alcohol (HLB value 12.5), POE (10) lanolin alcohol (HLB value 15.5), POE (20) lanolin alcohol (HLB value 16.0), POE (40) lanolin alcohol (HLB value 17.0), POE (20) sorbitol beeswax (HLB value 9.5) and other polyoxyethylene lanolins, lanolin alcohols and beeswax derivatives.

POE (20) castor oil (HLB value 10.5), POE (40) castor oil (HLB value 12.5), POE (50) castor oil (HLB value 14.0), POE (60) castor oil (HLB value 14.0), POE (20) hydrogenated castor oil (HLB value 10.5), POE (30) hydrogenated castor oil (HLB value 11.0), POE (40) hydrogenated castor oil (HLB value 13.5), POE (60) hydrogenated castor oil (HLB value 14.0), POE (80) hydrogenated castor oil (HLB value 16.5), POE (40) hydrogenated castor oil (100) hydrogenated castor oil (HLB value 16.5) and other polyoxyethylene castor oils and hydrogenated castor oils.

POE (5) phytosterol (HLB value 9.5), POE (10) phytosterol (HLB value 12.5), POE (20) phytosterol (HLB value 15.5), POE (30) phytosterol (HLB value 18.0), POE (25) phytostanol (HLB value 14.5), POE (30) cholestanol (HLB value 17.0) and other polyoxyethylene sterols and hydrogenated sterols.

POE (2) lauryl ether (HLB value 9.5), POE (4.2) lauryl ether (HLB value 11.5), POE (9) lauryl ether (HLB value 14.5), POE (5.5) cetyl ether (HLB value 10.5), POE (7) cetyl ether (HLB value 11.5), POE (10) cetyl ether (HLB value 13.5), POE (15) cetyl ether (HLB value 15.5), POE (20) cetyl ether (HLB value 17.0), POE (23) cetyl ether (HLB value 18.0), POE (4) stearyl ether (HLB value 9.0), POE (20) stearyl ether (HLB value 18.0), POE (21) stearyl ether (HLB value 18.0), POE (7) oleyl ether (HLB value 10.5), POE (10) oleyl ether (HLB value 14.5), POE (15) oleyl ether (HLB value 16.0), POE (20) oleyl ether (HLB value 17.0), POE (50) oleyl ether (HLB value 18.0), POE (10) behenyl ether (HLB value 10.0), POE (20) behenyl ether (HLB value 16.5), POE (30) behenyl ether (HLB value 18.0), POE (2) ($C_{12-15}$) alkyl ether (HLB value 9.0), POE (4) ($C_{12-15}$) alkyl ether (HLB value 10.5), POE (10) ($C_{12-15}$) alkyl ether (HLB value 15.5), POE (5) secondary alkyl ether (HLB value 10.5), POE (7) secondary alkyl ether (HLB value 12.0), POE (9) alkyl ether (HLB value 13.5), POE (12) alkyl ether (HLB value 14.5) and other polyoxyethylene alkyl ethers.

Polyoxypropylene 1 mol added (expressed as "POP (1)", same below), POP (4) cetyl ether (HLB value 9.5), POE (10) POP (4) cetyl ether (HLB value 10.5), POE (20) POP (8) cetyl ether (HLB value 12.5), POE (20) POP (6) decyltetradecyl ether (HLB value 11.0), POE (30) POP (6) decyltetradecyl ether (HLB value 12.0) and other polyoxyethylene polyoxypropylene alkyl ethers.

Polyethylene glycol 10 mol added (expressed as "PEG (10)", same below) monolaurate (HLB value 12.5), PEG (10) monostearate (HLB value 11.0), PEG (25) monostearate (HLB value 15.0), PEG (40) monostearate (HLB value 17.5), PEG (45) monostearate (HLB value 18.0), PEG (55) monostearate (HLB value 18.0), PEG 100 monostearate (HLB value 18.8), PEG 150 monostearate (HLB value 19), PEG (10) monooleate (HLB value 11.0), PEG distearate (HLB value 16.5), PEG diisostearate (HLB value 9.5) and other polyethylene glycol fatty acid esters.

PEG (8) glyceryl isostearate (HLB value 10.0), PEG (10) glyceryl isostearate (HLB value 10.0), PEG (15) glyceryl isostearate (HLB value 12.0), PEG (20) glyceryl isostearate (HLB value 13.0), PEG (25) glyceryl isostearate (HLB value 14.0), PEG glyceryl (30) isostearate (HLB value 15.0), PEG (40) glyceryl isostearate (HLB value 15.0), PEG (50) glyceryl isostearate (HLB value 16.0), PEG (60) glyceryl isostearate (HLB value 16.0) and other polyoxyethylene glyceryl isostearates.

Among these, polyoxyethylene adducts (=POE adducts) and polyethylene glycol adducts (=PEG adducts) are preferable. Particularly, PEG (10 to 150) monostearates are the most preferable from the viewpoint of stability and useability (affinity with the skin and lack of stickiness).

The ingredient (d) acts to emulsify the oil phase (inner phase) including the ingredient (a) and ingredient (b). The ingredient (d) may be used alone or in combinations of two or more types. The formulation amount of the ingredient (d) is preferably 0.5 to 8.0 mass%, more preferably 1.0 to 5.0 mass%, in the total mass of the cosmetic composition. If less than 0.5 mass%, the stability of the system tends to become poor, while even if formulated more than 5.0 mass%, not only is an effect commensurate with the increase in the formulation amount not seen, but also conversely stickiness tends to occur.

Further, in the present invention, the ingredient (d) is more preferably a combination of one having an HLB value of 9 to less than 15 and one having an HLB value of 15 or more. In this case, the one having an HLB value of 9 to less than 15 and the one having an HLB value of 15 or more are preferably combined in a total amount is 5 mass% or less at a ratio of 6/1 to 1/6 (mass ratio).

Note that, in the present invention, a low HLB value surfactant having an HLB value of less than 9 can also be used in a range not impairing the effect and stability of the present invention. As surfactants having an HLB value of less than 9, the following can be mentioned. However, the present invention is not limited to these examples.

POE (2) cetyl ether (HLB value 5), POE (3) cetyl ether (HLB value 6), POE (5) cetyl ether (HLB value 8) and other polyoxyethylene cetyl ethers.

POE (3) oleyl ether (HLB value 6), POE (5) oleyl ether (HLB value 8), POE (6) oleyl ether (HLB value 8) and other polyoxyethylene oleyl ethers.

POE (5) isocetyl ether (HLB value 8) and other polyoxyethylene isocetyl ethers.

POE (5) isostearyl ether (HLB value 8) and other polyoxyethylene isostearyl ethers.

POE (5) octyldodecyl ether (HLB value 7) and other polyoxyethylene octyldodecyl ethers.

POE (5) behenyl ether (HLB value 7) and other polyoxyethylene behenyl ethers.

POE (5) decyltetradecyl ether (HLB value 6) and other polyoxyethylene decyltetradecyl ethers.

POE (5) cholesteryl ether (HLB value 7) and other polyoxyethylene cholesteryl ethers.

POP (2) POE (3) decylether (HLB value 7) and other polyoxyethylene and polyoxypropylene decylethers.

Sorbitan sesquioleate (HLB value 7), sorbitan sesquiisostearate (HLB value 7) and other sorbitan fatty acid esters.

Glyceryl stearate (HLB value 5), self-emulsification type glyceryl stearate (HLB value 5), self-emulsification type glyceryl stearate (HLB value 6), self-emulsification type glyceryl stearate (HLB value 7), glyceryl isostearate (HLB

value 6), glyceryl diisostearate (HLB value 3) and other glyceryl mono-fatty acid esters.

Propylene glycol stearate (HLB value 4), propylene glycol laurate (HLB value 5), propylene glycol distearate (HLB value 2), propylene glycol dioleate (HLB value 2), propylene glycol dilaurate (HLB value 2), propylene glycol diisostearate (HLB value 2) and other propylene glycol fatty acid esters.

Glycol stearate (HLB value 4), glycol dilaurate (HLB value 2), glycol dioleate (HLB value 2), glycol distearate (HLB value 2), glycol fatty acid (C14-18) (HLB value 2) and other ethylene glycol fatty acid esters.

PEG (2) stearate (HLB value 5), PEG (3) stearate (HLB value 7), PEG (5) stearate (HLB value 8) and other polyethylene glycol monostearates.

PEG (3) oleate (HLB value 7) and other polyethylene glycol monooleates.

PEG (5) hydrogenated castor oil (HLB value 5), PEG (7) hydrogenated castor oil (HLB value 6), PEG (10) hydrogenated castor oil (HLB value 7) and other polyoxyethylene hydrogenated castor oils.

PEG (3) isostearate (HLB value 7) and other polyethylene glycol isostearates.

POE (3) cetyl ether stearate (HLB value 3), POE (4) cetyl ether stearate (HLB value 4), POE (6) cetyl ether stearate (HLB value 6), POE (7) cetyl ether stearate (HLB value 7) and other polyoxyethylene cetyl ether stearates.

POE (4) stearyl ether stearate (HLB value 4), POE (6) stearyl ether stearate (HLB value 5), POE (9) stearyl ether stearate (HLB value 6), POE (7) stearyl ether stearate (HLB value 7), POE (10) stearyl ether stearate (HLB value 7), POE (12) stearyl ether stearate (HLB value 8) and other polyoxyethylene stearyl ether stearates.

POE (3) lauryl ether stearate (HLB value 3), POE (5) lauryl ether stearate (HLB value 5), POE (8) lauryl ether stearate (HLB value 7), POE (10) lauryl ether stearate (HLB value 8) and other polyoxyethylene lauryl ether stearates.

POE (2) lauryl ether isostearate (HLB value 2), POE (5) lauryl ether isostearate (HLB value 5), POE (8) lauryl ether isostearate (HLB value 7), POE (8) lauryl ether isostearate (HLB value 7), POE (10) lauryl ether isostearate (HLB value 8) and other polyoxyethylene lauryl ether isostearates.

PEG (2) dilaurate (HLB value 4), PEG (3) dilaurate (HLB value 5), PEG (4) dilaurate (HLB value 5), PEG (6) dilaurate (HLB value 5), PEG (8) dilaurate (HLB value 8) and other polyethylene glycol dilaurates.

PEG (2) distearate (HLB value 2), PEG (3) distearate (HLB value 3), distearate PEG (3) (HLB value 3), PEG (3) distearate (HLB value 3), PEG (4) distearate (HLB value 4), PEG (6) distearate (HLB value 5), PEG (8) distearate (HLB value 6), PEG (12) distearate (HLB value 8), PEG (8) distearate (HLB value 8) and other polyethylene glycol distearates.

PEG (2) diisostearate (HLB value 3), PEG (3) diisostearate (HLB value 3), PEG (4) diisostearate (HLB value 4), PEG (6) diisostearate (HLB value 5), PEG (8) diisostearate (HLB value 6), PEG (12) diisostearate (HLB value 8) and other polyethylene glycol diisostearates.

PEG (2) dioleate (HLB value 3), PEG (3) dioleate (HLB value 3), PEG (4) dioleate (HLB value 4), PEG (6) dioleate (HLB value 5), PEG (8) dioleate (HLB value 6), PEG (12) dioleate (HLB value 8) and other polyethylene glycol dioleates.

PEG (4) sorbitol tetraoleate (HLB value 3), PEG (3) sorbitol tristearate (HLB value 3), PEG (4) sorbitan triisostearate (HLB value 3) and other polyoxyethylene sorbitol and sorbitan fatty acid esters.

PEG (3) glyceryl triisostearate (HLB value 2), PEG (5) glyceryl triisostearate (HLB value 3), PEG (10) glyceryl triisostearate (HLB value 3), PEG (20) glyceryl triisostearate (HLB value 8) and other polyoxyethylene glyceryl triisostearates.

PEG (10) glyceryl diisostearate (HLB value 7) and other polyoxyethylene glyceryl diisostearates.

PEG (3) glyceryl isostearate (HLB value 6), PEG (5) glyceryl isostearate (HLB value 8), PEG (6) glyceryl isostearate (HLB value 8) and other polyoxyethylene glyceryl isostearates.

PEG (3) glyceryl tristearate (HLB value 2), PEG (4) glyceryl tristearate (HLB value 2), PEG (5) glyceryl tristearate (HLB value 3), PEG (6) glyceryl tristearate (HLB value 3), PEG (10) glyceryl tristearate (HLB value 5), PEG (20) glyceryl tristearate (HLB value 8) and other polyoxyethylene glyceryl tristearates.

PEG (4) glyceryl distearate (HLB value 4) and other polyoxyethylene glyceryl distearates.

PEG (3) glyceryl trioleate (HLB value 2), PEG (5) glyceryl trioleate (HLB value 3), PEG (10) glyceryl trioleate (HLB value 5), PEG (20) glyceryl trioleate (HLB value 8) and other polyoxyethylene glyceryl trioleates.

PEG (5) hydrogenated castor oil isostearate (HLB value 4), PEG (10) hydrogenated castor oil isostearate (HLB value 5), PEG (15) hydrogenated castor oil isostearate (HLB value 7), PEG (20) hydrogenated castor oil isostearate (HLB value 8) and other polyoxyethylene hydrogenated castor oil isostearates.

PEG (5) hydrogenated castor oil triisostearate (HLB value 2), PEG (10) hydrogenated castor oil triisostearate (HLB value 4), PEG (15) hydrogenated castor oil triisostearate (HLB value 5), PEG (20) hydrogenated castor oil triisostearate (HLB value 6), PEG (30) hydrogenated castor oil triisostearate (HLB value 7), PEG (40) hydrogenated castor oil triisostearate (HLB value 8) and other polyoxyethylene hydrogenated castor oil triisostearates.

PEG (20) hydrogenated castor oil laurate (HLB value 8) and other polyoxyethylene hydrogenated castor oil laurates.

PEG (3) trimethylolpropane tristearate (HLB value 2), PEG (5) trimethylolpropane tristearate (HLB value 3),

PEG (10) trimethylolpropane tristearate (HLB value 5) and other polyoxyethylene trimethylolpropane tristearates.

PEG (3) trimethylolpropane trimyristate (HLB value 2), PEG (5) trimethylolpropane trimyristate (HLB value 3) and other polyoxyethylene trimethylolpropane trimyristates.

PEG (3) trimethylolpropane distearate (HLB value 3), PEG (4) trimethylolpropane distearate (HLB value 4), PEG (3) trimethylolpropane distearate (HLB value 4) and other polyoxyethylene trimethylolpropane distearates.

PEG (3) trimethylolpropane triisostearate (HLB value 2), PEG (20) trimethylolpropane triisostearate (HLB value 8) and other polyoxyethylene trimethylolpropane triisostearates.

Dihexyldecyl lauroyl glutamate (HLB value 3), dioctyldodecyl lauroyl glutamate (HLB value 3), dioctyldodecyl stearoyl glutamate (HLB value 3), dioctyldodecyl lauroyl glutamate (HLB value 3), deoctyl POE (2) dodecyl ether lauroyl glutamate (HLB value 4), dioctyl POE (5) dodecyl ether lauroyl glutamate (HLB value 6), POE (2) distearyl ether lauroyl glutamate (HLB value 4), POE (5) distearyl ether lauroyl glutamate (HLB value 7) and other N-acyl glutamic acid esters.

Hexyldecyl myristoyl methylaminopropionate (HLB value 4) and other N-acyl neutral amino acid esters.

Polyglyceryl (2) stearate (HLB value 6), polyglyceryl (2) stearate (HLB value 8), polyglyceryl (2) distearate (HLB value 4), polyglyceryl (3) distearate (HLB value 5), polyglyceryl (6) distearate (HLB value 8), polyglyceryl (10) tristearate (HLB value 8), polyglyceryl (2) diisostearate (HLB value 4), polyglyceryl (2) triisostearate (HLB value 3), polyglyceryl (3) diisostearate (HLB value 5), polyglyceryl (6) diisostearate (HLB value 8) polyglyceryl (10) triisostearate (HLB value 8), polyglyceryl (2) oleate (HLB value 8), polyglyceryl (2) diisostearate (HLB value 4), polyglyceryl (3) diisostearate (HLB value 5) and other polyglyceryl fatty acid esters.

PEG (7) dimethicone (HLB value 5), PEG (10) dimethicone (HLB value 6) and other silicone based surfactants.

In the oil-in-water emulsion type skin cosmetic composition of the present invention, the oil phase (i.e., inner phase) may contain, in addition to the above ingredient (a) and ingredient (b), various types of oil ingredients, oil soluble ingredients and the like. Further, the aqueous phase (i.e., outer phase) may contain, in addition to the ingredient (c) and the ingredient (d), various types of water soluble ingredients and the like. In the present invention, the aqueous phase (i.e., outer phase) is preferably 50.0 to 90.0 mass% based upon the total mass of the cosmetic composition. If the aqueous phase is less than 50.0 mass%, the weight is felt and stickiness occurs in some cases. On the other hand, if more than 90.0 mass%, while there is a clean feeling, the result is not moist and the effect of the present invention, that is, a feeling in use of moistness while clean becomes difficult to obtain.

In the oil-in-water emulsion type skin cosmetic composition of the present invention, other than the above ingredients, ingredients which can be formulated in general emulsion compositions can be appropriately formulated in a range which does not impede the effect of the present invention. As these kinds of ingredients, for example, ultraviolet absorbers, ultraviolet scatterers, waxes, hydrocarbon oils, silicone oils, polyhydric alcohols, water soluble polymers and the like can be mentioned, but the invention is not limited to these examples.

As ultraviolet absorbers, for example, para-aminobenzoic acid, octyl-p-methoxy-cinnamate (2-ethylhexyl-p-methoxy-cinnamate), glyceryl mono-2-ethylhexanoyl diparamethoxycinnamate, silyl isopentyl trimethoxycinnamate trisiloxane and other cinnamic acid based ultraviolet absorbers, 2,2'-hydroxy-5-methyl phenylbenzotriazole, 2-(2'-hydroxy-5'-t-octylphenyl)benzotriazole, 2-(2'-hydroxy-5'-methyl phenylbenzotriazole, 4-methoxy-4'-t-butyldibenzoylmethane, 5-(3,3-dimethyl-2-norbornylidene)-3-pentan-2-one, bis-ethylhexyloxyphenol-methoxyphenyl triazine, 2,4,6-tris[4-(2-ethylhexyloxycarbonyl)anilino]1,3,5- triazine, dimorpholinopyridazinone and the like can be mentioned.

As ultraviolet scatterers, for example, fine particles of titanium dioxide, fine particles of zinc oxide, fine particles of iron oxide, fine particles of cerium oxide and other powders of an average particle size of 10 to 100 nm can be mentioned.

Further, ultraviolet scatterers made hydrophobic by a silicone treatment by methyl hydrogen polysiloxane or a silane coupling agent, a metal soap treatment, a fluorine treatment by a perfluoroalkyl phosphoric acid diethanol amine salt or perfluoroalkylsilane, a dextrin fatty acid ester treatment or the like may also be suitably included, if necessary.

As waxes, for example, beeswax, Candelilla wax, Carnauba wax, lanolin, liquid lanolin, Jojoba wax and the like may be mentioned.

As hydrocarbon oils, for example, liquid paraffin, ozocerite, squalane, pristane, paraffin, ceresin, squalene, petrolatum, microcrystalline wax, polyethylene wax, Fischer-Tropsch wax and the like may be mentioned.

As silicone oils, for example, chain polysiloxanes (for example, dimethyl polysiloxane, methylphenyl polysiloxane, diphenyl polysiloxane and the like); ring polysiloxanes (for example, decamethyl cyclopentasiloxane, dodecamethyl cyclohexasiloxane and the like), silicone resins forming a three-dimensional network structure, silicone rubbers with an average molecular weight of 200,000 or more, various modifier polysiloxanes (amino modified polysiloxane, polyether modified polysiloxane, alkyl modified polysiloxanes, fluorine modified polysiloxanes and the like), and the like may be mentioned.

As polyhydric alcohols, for example, polyethylene glycol, glycerin, diglycerin, 1,3-butylene glycol, erythritol, sorbitol, xylitol, maltitol, 1,2-pentanediol, hexylene glycol and the like may be mentioned.

As water soluble polymers, for example, carrageenan, pectin, mannan, curdlan, chondroitin sulfate, starch, glycogen, gum arabic, sodium hyaluronate, tragacanth gum, xanthan gum, mucoitin sulfuric acid, hydroxyethyl guar gum, carboxymethyl guar gum, guar gum, dextran, keratan sulfate, locust bean gum, succinoglucan, chitin, chitosan,

carboxymethyl chitin, agar and the like may be mentioned.

As others, ethanol and other lower alcohols; butylhydroxy toluene, δ-tocopherol, phytin and other antioxidants; benzoic acid, salicylic acid, sorbic acid, p-hydroxybenzoic acid alkyl esters, phenoxy ethanol, hexachlorophen, poly(ε-lysine) and other preservatives; citric acid, lactic acid, hexametaphosphoric acid and other organic or inorganic acids and their salts; vitamin A, vitamin A palmitate, vitamin A acetate and other vitamin A derivatives, vitamin B6 hydrochloride, vitamin B6 tripalmitate, vitamin B6 dioctanoate, vitamin B2 and their derivatives, vitamin B12, vitamin B15 and their derivatives and other vitamin B's, α-tocopherol, β-tocopherol, vitamin E acetate and other vitamin E's, vitamin D's, vitamin H, pantothenic acid, pantethine and other vitamins; γ-oryzanol, allantoin, glycyrrhizinic acid (salt), glycyrrhetinic acid stearyl glycyrrhetinate, hinokitiol, bisabolol, eukalypton, thymol, inositol, saikosaponin, carrot saponin, sponge gourd saponin, mukurossi peel saponin and other saponins, pantothenyl ethyl ether, arbutin, cepharanthine and other various medicines, extracts of plants such as curly dock, sophora, Nuphar, orange, sage, yarrow, mallow, swertia herb, thyme, Japanese angelica root, bitter orange peel, white birch, horsetail, sponge gourd, horse chestnut, saxifrage, scutellaria root, arnica, lily, mugwort, peony root, aloe, gardenia, and cherry leaf, β-carotene and other colors and the like may also be formulated.

As the oil-in-water emulsion type skin cosmetic composition of the present invention, there are milky emulsions, skin creams, hair creams, liquid foundations, eye liners, mascaras, eye shadows and other milky liquid or creamy products. These products can be produced by ordinary methods by formulating the above essential ingredients and ingredients ordinarily formulated into these cosmetic compositions.

## Sunscreen Cosmetic

For the cosmetic composition of the present invention, as the ingredient (a), one or both of the isononyl 2-ethylhexanoate represented by the formula (I) and the 2-ethylhexyl 2-ethylhexanoate represented by the formula (II) may be used.

In the present invention, in particular the esters having the above formulae (I) and (II) are selected and used from esters of 2-ethylhexanoic acid (i.e., carbon chain length=8) having shorter carbon chain lengths than the above conventionally used esters of isononanoic acid. These two esters have respective molecular weights of 270 and 256 and are included in the class of smallest molecular weights in the ester oils which can be conventionally used for cosmetic compositions from the viewpoint of safety. The molecular weights are smaller even compared to the isononanoate esters widely used in conventional external preparations for the skin. By that extent, in useability, the cosmetic composition is clean and fresh in feel, is free from stickiness, and further is superior in affinity with the skin. Further, they are superior to isononanoate esters in the uniformity of the oil phase in a combined system of a high polarity oil containing an ultraviolet absorber and a nonpolar oil containing silicone oil. Furthermore, they are superior in the dispersion and stabilization of an ultraviolet scatterer of zinc oxide and titanium dioxide to isononanoate esters.

The amount of the ingredient (a) formulated in the sunscreen cosmetic of the present invention is preferably made a lower limit value of 1 mass% or more, more preferably 3 mass% or more, particularly preferably 5 mass% or more. Further, the upper limit value is preferably made 60 mass% or less, more preferably 40 mass% or less, particularly preferably 30 mass% or less. If the formulation amount is less than 1 mass%, the effect in emulsion stability, dispersibility and useability is difficult sufficiently obtain, while even if formulated more than 60 mass%, an effect commensurate with the increase in the formulation amount cannot be obtained.

As the ultraviolet absorber of the ingredient (b), those generally used in cosmetic compositions may be broadly mentioned. While they are not particularly limited, preferably p-aminobenzoic acid derivatives, salicylic acid derivatives, cinnamic acid derivatives, β,β-diphenyl acrylate derivatives, benzophenone derivatives, benzylidene camphor derivatives, phenylbenzimidazole derivatives, triazine derivatives, phenylbenzotriazole derivatives, anthranil derivatives, imidazoline derivatives, benzal malonate derivatives, 4,4-diarylbutadiene derivatives, and the like can be illustrated. However, the invention is not limited to these.

As the above p-aminobenzoic acid derivatives, p-aminobenzoic acid (abbreviated as "PABA" hereinbelow), ethyl PABA, ethyl dihydroxypropyl PABA, ethyl hexyldimethyl PABA (e.g., "Escalol 507"; ISP), glyceryl PABA, PEG-25-PABA (e.g., "Uvinul P 25"; BASF), and the like may be illustrated.

As the above salicylic acid derivatives, homosalate (e.g., "Eusolex HMS"; Rona/EM Industries Inc.), ethylhexyl salicylate (e.g., "Neo Heliopan OS"; Haarmann and Reimer Ltd.), dipropylene glycolsalicylate (e.g., "Dipsal"; Scher Chemicals Inc.), TEA salicylate (e.g., "Neo Heliopan TS"; Haarmann and Reimer Ltd.) and the like may be illustrated.

As the above cinnamic acid derivatives, ethylhexyl methoxycinnamate (e.g., "Parsol MCX"; Hoffmann-La Roche Inc.), isopropyl methoxycinnamate, isoamyl methoxycinnamate (e.g., "Neo Heliopan E1000"; Haarmann and Reimer Ltd.), cinoxate, DEA methoxycinnimate, diisopropyl methylcinnimate, glycerylethyl hexanoate-dimethoxycinnimate and the like may be illustrated.

As the above β,β-diphenyl acrylate derivative, octocrylene (e.g., "Uvinul N539"; BASF), etocrylene (e.g., "Uvinul N35"; BASF) and the like can be illustrated.

As the above benzophenone derivative, benzophenone 1 (e.g., "Uvinul 400"; BASF), benzophenone 2 (e.g., "Uvinul D50"; BASF), benzophenone 3 or oxybenzone (e.g., "Uvinul M40"; BASF), benzophenone 4 (e.g., "Uvinul MS40"; BASF), benzophenone 5, benzophenone 6 (e.g., "Helisorb 11"; Norquay Technology Inc.), benzophenone 8 (e.g., "Spectra-Sorb UV-24"; American Cyanamid Co.), benzophenone 9 (e.g., "Uvinul DS-49"; BASF), benzophenone 12 and the like may be illustrated.

As the above benzylidene camphor derivative, 3-benzylidene camphor (e.g. "Mexoryl SD"; Chimex Inc. Ltd.), 4-methyl benzylidene camphor, benzylidene camphor sulfonic acid (e.g., "Mexoryl SL"; Chimex Inc. Ltd.), camphor benzalkonium methosulfate (e.g., "Mexoryl SO"; Chimex Inc. Ltd.), terephthalylidene dicamphor sulfonic acid (e.g., "Mexoryl SX"; Chimex Inc. Ltd.), polyacrylamidemethyl benzylidene camphor (e.g., "Mexoryl SW"; Chimex Inc. Ltd.) and the like may be illustrated.

As the above phenylbenzimidazole derivative, phenylbenzimidazole sulfonic acid (e.g., "Eusolex 232"; Merck KGaA), disodium phenyl dibenzimidazole tetrasulfonate (e.g., "Neo Heliopan AP"; Haarmann and Reimer Ltd.) and the like may be illustrated.

As the above triazine derivative, aniso triazine (e.g., "Tinosorb S"; Ciba Specialty Chemicals Inc.), ethylhexyl triazone (e.g., "Uvinul T150"; BASF), diethylhexyl butamide triazone (e.g., "Uvasorb HEB"; 3V Sigma), 2,4,6-tris(diisobutyl-4'-aminobenzal malonate)-s-triazine and the like may be illustrated.

As the above phenylbenzotriazole derivative, drometrizole trisiloxane (e.g., "Silatrizole"; Rhodia Chimie Inc.), methylene bis(benzotriazolyl tetramethylbutylphenol) (e.g., sold in a micro form in a water based dispersion as "Tinosorb M" (Ciba Specialty Chemicals Inc.) or sold in a solid form as "Mixxim BB/100" (Fairmount Chemical Co. Inc.)) and the like may be illustrated.

As the above anthranil derivative, menthyl anthranilate (e.g., "Neo Heliopan MA"; Haarmann and Reimer Ltd.) and the like may be illustrated.

As the above imidazoline derivative, ethylhexyl dimethoxybenzylidene dioxoimidazoline propionate and the like may be illustrated.

As the above benzal malonate derivative, a polyorganosiloxane having a benzal malonate functional group (e.g., "Parsol SLX"; Hoffmann-La-Roche Inc.) and the like may be illustrated.

As the above 4,4-diarylbutadiene derivative, 1,1-dicarboxy(2,2'-dimethylpropyl)-4,4-diphenyl butadiene and the like may be illustrated.

Among these, particularly preferably ethylhexyl salicylate, ethylhexyl methoxycinnamate, octocrylene, benzophenone-3, benzophenone-4, benzophenone-5, 4-methyl benzylidene camphor, terephthalylidene dicamphor sulfonic acid, phenylbenzimidazole sulfonic acid, disodium phenyl dibenzimidazole tetrasulfonic acid, aniso triazine, ethylhexyl triazone, diethylhexyl butamide trizone, 2,4,6-tris(diisobutyl-4'-aminobenzal malonate)-s-triazine, drometrizole trisiloxane, methylene bis(benzotriazolyl tetramethylbutylphenol), 1,1-dicarboxy (2,2'-dimethylpropyl)-4,4-diphenylbutadiene, and their mixtures and the like can be mentioned. The ingredient (b) may be used alone or in combinations of two or more types.

The amount of the ingredient (b) formulated in the sunscreen cosmetic of the present invention is preferably made a lower limit value of 3 mass% or more, more preferably 5 mass% or more. Further, the upper limit value is preferably made 20 mass% or less, more preferably 15 mass% or less. If the amount formulated is less than 3 mass%, it is difficult to obtain a sufficient SPF value, while even if formulated more than 20 mass%, an effect commensurate with the increase of the formulated amount cannot be obtained.

As the ultraviolet scatterer of the ingredient (c), zinc oxide and titanium dioxide are preferably used.

Zinc Oxide

The zinc oxide used in the present invention is not particularly limited. The zinc oxides ordinarily used in cosmetic compositions may be broadly mentioned. Preferably, one more superior in dispersibility, for example, one treated on its surface by a known method, if necessary, can be used.

As a method of surface treatment, silicone treatment with methyl hydrogen polysiloxane, methyl polysiloxane and the like; fluorine treatment with perfluoroalkyl phosphate esters, perfluoro alcohols and the like; amino acid treatment with N-acyl glutamic acid and the like; lecithin treatment; metal soap treatment; fatty acid treatment; alkyl phosphate ester treatment; and the like can be mentioned. Among these, zinc oxide treated on its surface with silicone is preferred.

The silicone usable in the surface treatment is not particularly limited, but, for example, methyl polysiloxane, methylphenyl polysiloxane, methyl hydrogen polysiloxane, methyl cyclopolysiloxane, octamethyl cyclotetrasiloxane, decamethyl cyclopentasiloxane, dodecamethyl cyclohexasiloxane, octamethyl trisiloxane, tetradecamethyl hexasiloxane, dimethylsiloxane-methyl(polyoxyethylene)siloxane-methyl(polyoxypropylene)siloxane copolymer, dimethylsiloxane-methyl(polyoxyethylene)siloxane copolymer, dimethylsiloxane-methyl(polyoxypropylene) siloxane copolymer, dimethylsiloxane-methylcetyloxysiloxane copolymer, dimethylsiloxane-methylstearoxysiloxane copolymer and other various silicone oils may be mentioned. Preferably, it is methyl hydrogen polysiloxane. The use of such silicone-treated

zinc oxide is useful for the provision of a superior sunscreen cosmetic rich in water repellency, ultraviolet blocking, transparency, adhesion and dispersibility.

Paricularly, when the cosmetic composition of the present invention is a water-in-oil emulsion type sunscreen cosmetic composition, a zinc oxide treated on its surface in this way is preferably used. On the other hand, when an oil-in-water type oxidizing emulsion sunscreen cosmetic, one not treated on its surface to make it hydrophobic is preferably used.

The amount of silicone usable in the surface treatment of zinc oxide is usually 1 to 20 mass% per 100 mass% of silicone-treated zinc oxide, preferably 2 to 14 mass%, more preferably 2 to 10 mass%, particularly 2 to 5 mass%. The method of silicone treatment with zinc oxide is not particularly limited. It is possible to suitably select a conventional known method.

If giving specific examples of the zinc oxide usable in the present invention, MZ-300 (no surface treatment agent, particle size 30 to 40 nm, made by Tayca Corporation), MZ-303S (methicone treatment, particle size 30 to 40 nm, made by Tayca Corporation), MZ-303M (dimethicone treatment, particle size 30 to 40 nm, made by Tayca Corporation), MZ-500 (no surface treatment agent, particle size 20 to 30 nm, made by Tayca Corporation), MZ-505S (methicone treatment, particle size 20 to 30 nm, made by Tayca Corporation), MZ-505M (dimethicone treatment, particle size 20 to 30 nm, made by Tayca Corporation), MZ-700 (no surface treatment agent, particle size 10 to 20 nm, made by Tayca Corporation), MZ-707S (methicone treatment, particle size 10 to 20 nm, made by Tayca Corporation), FINEX-25 (no surface treatment agent, particle size 60 nm, made by Sakai Chemical Industry Co. Ltd.), FINEX-25LP (dimethicone treatment, particle size 60 nm, made by Sakai Chemical Industry Co. Ltd.), FINEX-50 (no surface treatment agent, particle size 20 nm, made by Sakai Chemical Industry Co. Ltd.), FINEX-50LP (dimethicone treatment, particle size 20 nm, made by Sakai Chemical Industry Co. Ltd.), FINEX-75 (no surface treatment agent, particle size 10 nm, made by Sakai Chemical Industry Co. Ltd.) and the like may be mentioned. However, the invention is not limited to these examples.

The zinc oxide usable in the present invention preferably has an average primary particle size of 40 nm or less, more preferably 30 nm or less. This is because, if the average primary particle size is much greater than 40 nm, a tendency to cause graininess and residue is observed. The lower limit value of the average primary particle size is not particularly limited, but the smaller the particle size, the more expensive it becomes, and therefore considering economic efficiency, 5 nm or more is good, preferably 10 nm or more.

Note that in the present invention, the "average primary particle size" is not particularly limited and means the size of the primary particles measured by a method generally used for zinc oxide and titanium dioxide. Specifically, it is found from a transmission electron micrograph as the arithmetical mean of the major axis and minor axis of the particles.

The particles are not particularly limited in form. They may be in the state of primary particles or may form agglomerated secondary aggregates. Further, they are not particularly limited in the type of shape such as spheres, elliptical shapes, fractured forms, etc.

A more preferable embodiment of the silicone treated zinc oxide in the present invention is dimethyl hydrogen polysiloxane treated zinc oxide (surface treatment of 1 to 5 mass%) having an average primary particle size of 5 to 40 nm.

<u>Titanium Dioxide</u>

The titanium dioxide usable in the present invention is not particularly limited. Those usually used in cosmetic compositions may be widely mentioned. The crystalline form of the titanium dioxide is not particularly an issue. It may be anatase, rutile or brookite.

Preferably, it is one with a higher ultraviolet blocking power. As such ones, for example, to increase the ultraviolet dispersion effect, those treated on the surface may be mentioned. As such a surface treatment method, it is possible to use any treatment being used by ordinary cosmetic compositions, without particular limitation. As this kind of treatment, for example, a method making the surface of the titanium dioxide adsorb oils and fats, a fatty acid treatment method of using a fatty acid to treating titanium dioxide esterified or etherified utilizing hydroxy groups and other functional groups, a metal soap treatment method using an aluminum salt or zinc salt of a fatty acid such as aluminum stearate or zinc stearate, instead of a fatty acid in the above method, a silicone treatment method using methyl polysiloxane or methyl hydrogen polysiloxane, instead of a fatty acid, and further a method treating it with a fluorine compound having a perfluoroalkyl group instead of a fatty acid and the like may be mentioned.

Preferably, it is aluminum stearate treated titanium dioxide treated with aluminum stearate. The ratio of the aluminum stearate contained in the aluminum stearate treated titanium dioxide is not particularly limited, but 1 to 20 mass% may be mentioned. Further, as the ratio of the titanium dioxide, 80 to 99 mass% may be mentioned as a preferable formulation amount.

In particular, when the cosmetic composition of the present invention is a water-in-oil emulsion type sunscreen cosmetic composition, titanium dioxide treated on its surface in this way is preferably used. On the other hand, when it is an oil-in-water type oxidizing emulsion sunscreen cosmetic composition, one not treated on its surface to make it hydrophobic is preferably used.

As a specific example of the titanium dioxide usable in the present invention, Tipaque CR-50 (rutile type, aluminum oxide treatment, particle size 25 nm, made by Ishihara Sangyou Kabushiki Kaisha Ltd.), Bayertitan R-KB-1 (rutile type, zinc oxide treatment, aluminum oxide treatment, silica dioxide treatment, particle size 30 to 40 nm, made by Bayer AG), Tipaque TTO-M-1 (rutile type, zirconium oxide and aluminum oxide treatment, particle size 10 to 25 nm, made by Ishihara Sangyou Kabushiki Kaisha Ltd.), Tipaque TTO-D-1 (rutile type, zirconium oxide treatment, aluminum oxide treatment, particle size 20 to 3 nm, made by Ishihara Sangyou Kabushiki Kaisha Ltd.), Tipaque A-100 (anatase type, no surface treatment, particle size 0.4 μm, made by Ishihara Sangyou Kabushiki Kaisha Ltd.), Kronos KA-10 (anatase type, no treatment, particle size 0.3 to 0.5 μm, made by Titan Kogyo K.K.), Kronos KA-15 (anatase type, no treatment, particle size 0.3 to 0.5 μm, made by Titan Kogyo K.K.), Kronos KA-20 (anatase type, aluminum oxide treatment, particle size 0.3 to 0.5 μm, made by Titan Kogyo K.K.), Kronos KA-30 (anatase type, no treatment, particle size 0.2 to 0.4 μm, made by Titan Kogyo K.K.), Kronos KA-35 (anatase type, no treatment, particle size 0.2 to 0.4 μm, made by Titan Kogyo K.K.), Kronos KA-80 (anatase type, aluminum oxide treatment, silica dioxide treatment, particle size 0.3 to 0.5 μm, made by Titan Kogyo K.K.), Kronos KR-310 (rutile type, no treatment, particle size 0.3 to 0.5 μm, made by Titan Kogyo K.K.), Kronos KR-380 (rutile type, aluminum oxide treatment, silica dioxide treatment, particle size 0.3 to 0.5 μm, made by Titan Kogyo K.K.), Kronos KR-460 (rutile type, aluminum oxide treatment, particle size 0.2 to 0.4 μm, made by Titan Kogyo K.K.), Kronos KR-480 (rutile type, aluminum oxide treatment, silica dioxide treatment, particle size 0.2 to 0.4 μm, made by Titan Kogyo K.K.), Kronos KR-270 (rutile type, zinc oxide treatment, aluminum oxide treatment, particle size 0.2 to 0.4 μm, made by Titan Kogyo K.K.), Titanix JR-301 (rutile type, aluminum oxide treatment, particle size 0.3 μm, made by Tayca K.K.), Titanix JR-403 (rutile type, aluminum oxide treatment, silica dioxide treatment, particle size 0.25 μm, made by Tayca K.K.), Titanix JR-405 (rutile type, aluminum oxide treatment, particle size 0.21 μm, made by Tayca K.K.), Titanix JR-600A (rutile type, aluminum oxide treatment, particle size 0.25 μm, made by Tayca K.K.), Titanix JR-605 (rutile type, aluminum oxide treatment, particle size 0.25 μm, made by Tayca K.K.), Titanix JR-600E (rutile type, aluminum oxide treatment, particle size 0.27 μm, made by Tayca K.K.), Titanix JR-603 (rutile type, aluminum oxide treatment, zirconium oxide treatment, particle size 0.28 μm, made by Tayca K.K.), Titanix JR-805 (rutile type, aluminum oxide, silica dioxide treatment, particle size 0.29 μm, made by Tayca K.K.), Titanix JR-806 (rutile type, aluminum oxide treatment, silica dioxide treatment, particle size 0.25 μm, made by Tayca K.K.), Titanix JR-701 (rutile type, aluminum oxide treatment, silica dioxide treatment, zinc oxide treatment, particle size 0.27 μm, made by Tayca K.K.), Titanix JRNC (rutile type, aluminum oxide treatment, silica dioxide treatment, zirconium oxide treatment, made by Tayca K.K.), Titanix JR-800 (rutile type, aluminum oxide treatment, silica dioxide treatment, made by Tayca K.K.), Titanix JR (rutile type, no treatment, particle size 0.27 μm, made by Tayca K.K.), Titanix JA-1 (anatase type, no treatment, particle size 0.18 μm, made by Tayca K.K.), Titanix JA-C (anatase type, no treatment, particle size 0.18 μm, made by Tayca K.K.), Titanix JA-3 (anatase type, no treatment, particle size 0.18 μm, made by Tayca K.K.), Titanix JA-4 (anatase type, aluminum oxide treatment, particle size 0.18 μm, made by Tayca K.K.), Titanix JA-5 (anatase type, no treatment, particle size 0.18 μm, made by Tayca K.K.) and the like can be mentioned. However, the invention is not limited to these examples.

Further, to increase the ultraviolet dispersion effect, the titanium dioxide may be prepared in a fine particle form. The fine particle titanium dioxide is not limited, but preferably one with an average primary particle size of 30 nm or less, more preferably 20 nm or less, can be mentioned. If the average primary particle size is greatly over 30 nm, it tends to cause graininess and residue. The lower limit value of the average primary particle size is not particularly limited, but the smaller the particle size, the more expensive it becomes, so, if considering economic efficiency, 5 nm or more is good, preferably 10 nm or more.

The total amount of the ingredient (c) formulated in the sunscreen cosmetic composition of the present invention is preferably made a lower limit value of 0.5 mass% or more, more preferably 1 mass% or more, particularly 3 mass% or more. Further, the upper limit value is preferably made 50 mass% or less, more preferably 40 mass% or less. If the formulation amount is less than 0.5 mass%, a sufficient SPF value is difficult to obtain, while even if formulated more than 50 mass%, an effect commensurate with the increase of the formulation amount cannot be obtained and preparation becomes difficult. Particularly, when using zinc oxide and titanium dioxide as the ingredient (c), if the amount formulated becomes excessive, problems occur in respect to useability such as slip on the skin and graininess or in respect to stability, and therefore this is not preferable.

Note that when blending in zinc oxide as the ingredient (c), it is preferable to formulate it in the range of 5 to 40 mass%. Further, when formulating in titanium dioxide, it is preferable to blend it in the range of 0.5 to 10 mass%.

As the silicone oil of the ingredient (d), ones generally used in cosmetic compositions may be widely mentioned. Specifically, methyl polysiloxane, octamethyl siloxane, decamethyl tetrasiloxane, methyl hydrogen polysiloxane, methylphenyl polysiloxane, hexamethyl cyclotrisiloxane, octamethyl cyclotetrasiloxane, decamethyl cyclopentasiloxane and the like may be illustrated. Preferably, octamethyl cyclotetrasiloxane, decamethyl cyclopentasiloxane and the like may be illustrated. However, the invention is not limited to these examples. The ingredient (d) may be used alone or in combinations of two or more types.

The amount of the ingredient (d) formulated in the sunscreen cosmetic composition of the present invention is preferably made a lower limit value of 1 mass% or more, more preferably 3 mass% or more, particularly preferably 5

mass% or more. Further, the upper limit value is preferably 70 mass% or less, more preferably 50 mass% or less, particularly preferably 30 mass% or less. If the formulation amount is less than 1 mass%, the above trend is observed, while even if formulated more than 70 mass%, an effect commensurate with the increase of the formulated amount is not obtained and further graininess is caused and the ultraviolet protective effect deteriorates.

The cosmetic composition of the present invention has the above ingredients (a) to (d) as essential ingredients. Further, it may contain, as the ingredient (e), one or more ingredients selected from silicone based surfactants, glycerin or polyglyceryl fatty acid esters from the viewpoint of the improvement in the useability and stability.

Silicone Based Surfactant

The silicone based surfactant is not particularly limited, but, for example, nonionic silicone based surfactants modified in the siloxane structure with a polyether group, epoxy polyether group, hydroxy group, animo group, epoxy group and the like; cationic silicone based surfactants modified in the siloxane structure with an ammonium base; amphoteric silicone based surfactants modified in the siloxane structure by a sulfobetain group, etc. may be mentioned, but the invention is not limited to these.

By using a silicone based surfactant introducing a modifying group to a polysiloxane chain, the dispersibility and stability of the titanium dioxide and zinc oxide in the preparations can be significantly improved compared to the use of only 2-ethylhexanoate ester and, furthermore, a cosmetic composition superior in the preparation ability can be prepared.

Preferably, in the point that adjustment of the polarity is possible across a wide range from hydrophilicity to lipophilicity, polyether modified silicone based surfactants may be mentioned. Among these, a poly(oxyethylene-oxypropylene)methyl polysiloxane copolymer, a polyoxyethylene methyl polysiloxane copolymer, a silicone chain branched type methyl polysiloxane copolymer, an alkyl chain branched type polyoxyethylene methyl polysiloxane copolymer, an alkyl chain-silicone chain branched type polyoxyethylene methyl polysiloxane copolymer, a cross-linked type polyoxyethylene methyl polysiloxane, a cross-linked type polyoxyethylene methyl polysiloxane containing an alkyl group, a branched type polyglycerin modified silicone, a cross-linked type polyglycerin modified silicone, a cross-linked type polyglycerin modified silicone containing an alkyl group and a branched type polyglycerin modified silicone containing an alkyl group can be preferably mentioned.

As the above poly(oxyethylene-oxypropylene) methyl polysiloxane copolymer, PEG/PPG-20/22 butylether dimethicone ("KF-6012"; made by Shin-Etsu Chemical Co. Ltd.), PEG/PPG-20/20 dimethicone ("BY22-008M"; made by Dow Corning Toray Silicones Co. Ltd.), lauryl PEG/PPG-18 methicone ("5200 Formulation Aid"; made by Dow Corning Toray Co. Ltd.), PEG/PPG-19/19 dimethicone ("5330 Fluid"; made by Dow Corning Toray Co. Ltd.), PEG/PPG-15/15 dimethicone ("5330 Fluid"; made by Dow Corning Toray Co. Ltd.) and the like may be mentioned.

As the polyoxyethylene methyl polysiloxane copolymer, PEG-11 methyl ether dimethicone ("KF-6011"; made by Shin-Etsu Chemical Co. Ltd.), PEG-9 dimethicone ("KF-6013"; made by Shin-Etsu Chemical Co. Ltd.), PEG-3("KF-6015"; made by Shin-Etsu Chemical Co. Ltd.), PEG-9 methyl ether dimethicone ("KF-6016"; made by Shin-Etsu Chemical Co. Ltd.), PEG-10 dimethicone ("KF-6017"; made by Shin-Etsu Chemical Co. Ltd.), PEG-11 methyl ether dimethicone ("KF-6018"; made by Shin-Etsu Chemical Co. Ltd.), PEG-9 dimethicone ("KF-6019"; made by Shin-Etsu Chemical Co. Ltd.), PEG-12 dimethicone ("SH3771M", "SH3772M", "SH3773M", "SH3775M" and the like made by Dow Corning Toray Co. Ltd.) and the like may be mentioned.

As the silicone chain branched type methyl polysiloxane copolymer, PEG-9 polydimethylsiloxyethyl dimethicone ("KF-6028"; made by Shin-Etsu Chemical Co. Ltd.) may be mentioned.

As the alkyl chain branched type polyoxyethylene methyl polysiloxane copolymer, PEG/PPG-10/3 oleyl ether dimethicone ("KF-6026"; made by Shin-Etsu Chemical Co. Ltd.) and the like may be mentioned.

As an alkyl chain-silicone chain branched type polyoxyethylene methyl polysiloxane copolymer, lauryl PEG-9 polydimethylsiloxyethyl dimethicone ("KF-6038"; made by Shin-Etsu Chemical Co. Ltd.) and the like may be mentioned.

As a cross-linked type polyoxyethylene methylpolysiloxane, a dimethicone (dimethicone/(PEG-10/15)) cross polymer ("KSG-210"; made by Shin-Etsu Chemical Co. Ltd.), a cyclomethicone PEG-12 dimethicone dimethicone cross polymer ("9011 Silicone Elastomer Blend"; made by Dow Corning Toray Silicones Co. Ltd.) may be mentioned.

As a cross-linked type polyoxyethylene methyl polysiloxane containing an alkyl base, a mineral oil-PEG-15 lauryl dimethicone cross polymer ("KSG-310"; made by Shin-Etsu Chemical Co. Ltd.), isododecane PEG-15 lauryl dimethicone cross polymer ("KSG-320"; made by Shin-Etsu Chemical Co. Ltd.), trioctanoin PEG-15 lauryl dimethicone cross polymer ("KSG-330"; made by Shin-Etsu Chemical Co. Ltd.), squalane PEG-15 lauryl dimethicone cross polymer-PEG-10 lauryl dimethicone cross polymer ("KSG-340"; made by Shin-Etsu Chemical Co. Ltd.) and the like may be mentioned.

As a branched type polyglycerin modified silicone, polyglyceryl 3-disiloxane dimethicone ("KF-6100"; made by Shin-Etsu Chemical Co. Ltd.), polyglyceryl 3-polydimethylsiloxy dimethicone ("KF-6104"; made by Shin-Etsu Chemical Co. Ltd.) and the like may be mentioned.

As a cross-linked type polyglycerin modified silicone, dimethicone-(dimethicone/polyglycerin 3) cross polymer ("KSG-710"; made by Shin-Etsu Chemical Co. Ltd.) and the like may be mentioned.

As a cross-linked type polyglycerin modified silicone containing an alkyl group, a mineral oil-(lauryl dimethicone/polyglycerin 3) cross polymer ("KSG-810"; made by Shin-Etsu Chemical Co. Ltd.), isododecane-(lauryl dimethicone/polyglycerin 3) cross polymer ("KSG-820; made by Shin-Etsu Chemical Co. Ltd."), trioctanoin-(lauryl dimethicone/polyglycerin 3) cross polymer ("KSG-830"; made by Shin-Etsu Chemical Co. Ltd.), squalane-(lauryl dimethicone/polyglycerin 3) cross polymer ("KSG-840"; made by Shin-Etsu Chemical Co. Ltd.) and the like may be mentioned.

As an alkyl group branched type polyglycerin modified silicone, lauryl polyglyceryl-3 polydimethylsiloxy dimethicone ("KF-6105"; made by Shin-Etsu Chemical Co. Ltd.) and the like may be mentioned.

Among these, a poly(oxyethylene-oxypropylene) methyl polysiloxane copolymer, alkyl chain branched type poly(oxyethylene·oxypropylene) methyl polysiloxane copolymer, silicone chain-alkyl chain branched type poly(oxyethylene-oxypropylene) methyl polysiloxane copolymer and the like may be preferably used.

<u>Glycerin or Polyglycerin Fatty Acid Esters</u>

As glycerin or polyglycerin fatty acid esters, glyceryl undecylenate, glyceryl myristate, glyceryl stearate, self-emulsification type glyceryl stearate, glyceryl isostearate, glyceryl oleate, glyceryl orivate, glyceryl dioleate, polyglyceryl-2 stearate, polyglyceryl-2 oleate, polyglyceryl-2 dioleate, polyglycery-2 isostearate, polyglyceryl-2 diisostearate, polyglyceryl-2 triisostearate, polyglyceryl-4 stearate, polyglyceryl-4 oleate, polyglyceryl-4 tristearate, polyglyceryl-4 pentaoleate, polyglyceryl-6 laurate, polyglyceryl-6 myristate, polyglyceryl-6 stearate, polyglyceryl-6 oleate, polyglyceryl-6 tristearate, polyglyceryl-6 tetrabehenate, polyglyceryl-6 pentastearate, polyglyceryl-6 pentaoleate, polyglyceryl-6 polyricinoleate, polyglyceryl-10 laurate, polyglyceryl-10 myristate, polyglyceryl-10 stearate, polyglyceryl-10 isostearate, polyglyceryl-10 oleate, polyglyceryl-10 linoleate, polyglyceryl-10 distearate, polyglyceryl-10 diisostearate, polyglyceryl-10 tristearate, polyglyceryl-10 trioleate, polyglyceryl-10 pentastearate, polyglyceryl-10 pentahydroxystearate, polyglyceryl-10 pentaisostearate, polyglyceryl-10 pentaoleate, polyglyceryl-10 heptastearate, polyglyceryl-10 heptaoleate, polyglyceryl-10 decastearate, polyglyceryl-10 decaisostearate, polyglyceryl-10 decaoleate, polyglyceryl-10 decamacadamiate, polyglyceryl-10 polyricinoleate and the like may be mentioned.

The cosmetic of the present invention can take either of an oil-in-water emulsion type or a water-in-oil emulsion type form. When made an oil-in-water emulsion type sunscreen cosmetic composition, a polyoxyethylene methyl polysiloxane copolymer, glycerin or polyglycerin fatty acid ester or the like is preferably used as the ingredient (e). Further, when made a water-in-oil emulsion type sunscreen cosmetic composition, an alkyl chain-silicone chain branched type polyoxyethylene methyl polysiloxane copolymer, silicone chain branched type polyoxyethylene methyl polysiloxane copolymer, polyoxyethylene methyl polysiloxane copolymer or the like is preferably used as the ingredient (e).

The amount of the ingredient (e) formulated in the sunscreen cosmetic composition of present invention is preferably made a lower limit value of 0.01 mass% or more, more preferably 0.1 mass% or more, particularly preferably 0.5 mass% or moree. Further, the upper limit value is preferably made 20 mass% or less, more preferably 10 mass% or less. If the formulation amount is less than 0.01 mass%, a tendency for the stability of the cosmetic to become poor is seen, while if the formulation amount is greatly more than 20 mass%, there is a tendency for stickiness to be caused and the useability to become bad.

The cosmetic composition of the present invention may have suitably formulated into it, in addition to the above ingredients, other ingredients usually used in cosmetic compositions if necessary. As such ingredients, water soluble polymers, oil soluble polymers, waxes, alcohols, hydrocarbon oils, fatty acids, higher alcohols, fatty acid esters, medicines and the like may be mentioned. However, the invention is not limited to these examples.

As water soluble polymers, a homopolymer or copolymers of 2-acrylamide-2-methylpropane sulfonic acid (abbreviated as "AMPS" hereinbelow) may be illustrated. The copolymers are copolymers including vinyl pyrrolidone, amide acrylate, sodium acrylate, hydroxyethyl acrylate and other comonomers. Namely, an AMPS homopolymer, vinyl pyrrolidone/AMPS copolymer, dimethylacrylamide/AMPS copolymer, amide acrylate/AMPS copolymer, sodium acrylate/AMPS copolymer and the like may be illustrated.

Furthermore, a carboxyvinyl polymer, ammonium polyacrylate, sodium polyacrylate, sodium acrylate/alkyl acrylate/sodium methacrylate/alkyl methacrylate copolymer, carrageenan, pectin, mannan, curdlan, chondroitin sulfate, starch, glycogen, gum arabic, sodium hyaluronate, tragacanth gum, xanthan gum, mucoitinsulfuric acid, hydroxyethyl guar gum, carboxymethyl guar gum, guar gum, dextran, keratan sulfate, locust bean gum, succinoglucan, chitin, chitosan, carboxymethyl chitin, agar and the like may be illustrated.

As an oil soluble polymer, trimethylsiloxy silicate, alkyl modified silicone, polyamide modified silicone, dimethicone cross polymer, (dimethicone/vinyl dimethicone) cross polymer, polymethyl silsesquioxane and the like may be illustrated.

As waxes, for example, beeswax, Candelilla wax, Carnauba wax, lanolin, liquid lanolin, Jojoba wax and the like may be illustrated.

As alcohols, ethanol, isopropanol and other lower alcohols, isostearyl alcohol, octyl dodecanol, hexyl decanol and other higher alcohols, ethylene glycol, propylene glycol, 1,3-butylene glycol, dipropylene glycol, polybutylene glycol and other polyhydric alcohols and the like may be illustrated.
As hydrocarbon oils, liquid paraffin, ozocerite, squalane, pristane, paraffin, ceresin, squalene, petrolatum, microcrystalline wax, polyethylene wax, Fischer-Tropsch wax and the like may be illustrated.
As fatty acids, lauric acid, myristic acid, palmitic acid, stearic acid, behenic acid, arachidonic acid and the like may be illustrated.
As higher alcohols, lauryl alcohol, myristyl alcohol, cetyl alcohol, stearyl alcohol, behenyl alcohol, arachyl alcohol, batyl alcohol, chimyl alcohol, carnaubyl alcohol, ceryl alcohol, corianyl alcohol, myricyl alcohol, lacceryl alcohol, elaidyl alcohol, isostearyl glyceryl ether, octyl alcohol, triacontyl alcohol, selachyl alcohol, cetostearyl alcohol, oleyl alcohol, lanolin alcohol, hydrogenated lanolin alcohol, hexyl decanol, octyl decanol and the like may be illustrated.
As fatty acid esters, myristyl myristate, cetyl palmitate, cholesteryl stearate, beeswax fatty acid 2-octyldodecyl and the like may be illustrated.
As medicines, salts of L-ascorbic acid and its derivatives, dipotassium glycyrrhizinate, monoammonium glycyrrhizinate and other glycyrrhizinates and their derivatives, stearyl glycyrrhetinate and other glycyrrhetinates and their derivatives, allantoin, salts of tranexamic acid and its derivatives, salts of alkoxysalicylic acid and its derivatives, salts of glutathione and its derivatives, allantoin, azulene and the like may be illustrated.
The oil-in-water or water-in-oil emulsion type sunscreen cosmetic composition of the present invention is a milky liquid product or cream product. These products may be produced by an ordinary method which formulates the above-stated essential ingredients and ingredients which are usually formulated in cosmetic compositions.

EXAMPLES

The present invention will now be explained in further detail with reference to, but the present invention is not limited to, the following Examples. Note that the formulated amounts are all shown by mass%.

Example I-1

Each of the following oil ingredients was dropped on the skin at the inside of the forearm in an amount of 10 μl and the area ($cm^2$) after the elapse of 1 minute was measured. The results are shown in Table I-1.

Table I-1

| Oil ingredient | Area after elapse of 1 minute from dropping ($cm^2$) |
|---|---|
| Pentaerythrityl tetraoctanoate | 1.0 |
| Trioctanoin | 2.2 |
| Squalane | 2.4 |
| Cetyl 2-ethylhexanoate | 3.8 |
| Diethylhexyl succinate | 3.9 |
| Tripropylene glycol pivalate | 4.0 |
| Isononyl isononate | 4.5 |
| Isononyl 2-ethylhexanoate | 6.5 |
| 2-ethylhexyl 2-ethylhexanoate | 7.2 |

As clear from the results in Table I-1, it is confirmed that isononyl 2-ethylhexanoate and 2-ethylhexyl 2-ethylhexanoate have a higher spreading value on human skin and superior affinity with the skin compared to the other oil ingredients widely used in conventional cosmetics.
Note that isononyl 2-ethylhexanoate is not listed in any official compendiums. There is no example of it being formulated into a cosmetic composition, but the compound is not toxic and is safe.

Example I-2

The useability (i.e., affinity with the skin) by various types of ester oils formulated into oil-in-water type emulsion type cream systems was evaluated by the following:

Namely, each sample of the composition shown in the following Table I-2 was evaluated for "affinity with the skin" by the following evaluation criteria. The results are shown in Table I-2.

Evaluation Criteria

Very good: Feeling of affinity with the skin is high
Good: Affinity with the skin can be felt
Fair: Affinity with the skin can be felt somewhat
Poor: Affinity with the skin cannot be felt

Table I-2

| | Sample A | Sample B | Sample C | Sample D | Sample E |
|---|---|---|---|---|---|
| Ion exchanged water | Bal. | Bal. | Bal. | Bal. | Bal. |
| Dimethylacrylamide/AMPS/ methylene bisacrylamide cross polymer | 0.5 | 0.5 | 0.5 | 0.5 | 0.5 |
| Ethylene diamine tetraacetate trisodium salt | 0.01 | 0.01 | 0.01 | 0.01 | 0.01 |
| 1,3-butylene glycol | 7.0 | 7.0 | 7.0 | 7.0 | 7.0 |
| Ethyl paraben | 0.2 | 0.2 | 0.2 | 0.2 | 0.2 |
| Butyl paraben | 0.1 | 0.1 | 0.1 | 0.1 | 0.1 |
| $\alpha$-olefin oligomer | 5.0 | 5.0 | 5.0 | 5.0 | 5.0 |
| 2-ethylhexyl 2-ethylhexanoate | 10.0 | - | - | - | - |
| Isononyl 2-ethylhexanoate | - | 10.0 | - | - | - |
| 2-ethylhexyl isononate | - | - | 10.0 | - | - |
| Isononyl isononate | - | - | - | 10.0 | - |
| Cetyl 2-ethylhexanoate | - | - | - | - | 10.0 |
| PEG (40) monostearate | 0.5 | 0.5 | 0.5 | 0.5 | 0.5 |
| Cetostearyl glucoside | 0.1 | 0.1 | 0.1 | 0.1 | 0.1 |
| Stearyl alcohol | 0.7 | 0.7 | 0.7 | 0.7 | 0.7 |
| Behenyl alcohol | 2.5 | 2.5 | 2.5 | 2.5 | 2.5 |
| Dimethyl silicone oil | 5.0 | 5.0 | 5.0 | 5.0 | 5.0 |
| Decamethyl cyclopentasiloxane | 3.0 | 3.0 | 3.0 | 3.0 | 3.0 |
| Glycerin (85%) for cosmetics | 3.0 | 3.0 | 3.0 | 3.0 | 3.0 |
| Affinity with the skin | Very good | Good | Good | Fair | Fair to Poor |

As is clear from the results in Table I-2, it is understood that sample A and sample B containing 2-ethylhexyl 2-ethylhexanoate and isononyl 2-ethylhexanoate are superior in affinity with the skin compared to samples C, D and E containing the conventionally widely used ester oil. In particular, sample A has a remarkably superior effect of affinity with the skin compared to the conventional ester oils.

Examples I-3 to I-12 and Comparative Examples I-1 to I-4

Using each of the formulations shown in the following Tables I-3 to I-4, an oil-in-water emulsion type skin cosmetic composition, i.e., skin cream was produced by the following method.

Method of Production

(1) to (11) were uniformly mixed and dissolved (oil phase) at 70°C. On the other hand, (12) to (24) were uniformly

mixed and dissolved (aqueous phase) at 70°C. Next, the 70°C oil phase was gradually added to the aqueous phase held at 70°C and the two emulsified by a homo mixer. After ending emulsification, the resultant mixture was rapidly cooled down to 40°C or less to obtain the targeted oil-in-water emulsion type skin cream. The obtained cream (sample) was evaluated by the following test methods for stability and useability (slip on skin, affinity with the skin, stickiness, clean feeling, emollient sensation and firm feeling). The results are shown in Tables I-3 to I-4.

Stability Test

The sample was observed for appearance after being left to stand at 50°C for 1 month by visual and microscopic observation and was judged by the following evaluation criteria.

Evaluation Criteria

Good: Separation and crystal precipitation could not be observed at all
Fair: Separation and crystal precipitation could not be observed much at all
Poor: Separation of liquid phase (oil phase or aqueous phase) or crystal precipitation occurred

Usability (Slip on Skin)

A test of the feeling in use was performed by an expert panel consisting of women (10). The slip on the skin was evaluated by them by the following evaluation criteria:

Evaluation Criteria

Very good: All 10 women judged slip was light and smooth
Good: 7 to 9 women judged slip was light and smooth
Fair: 3 to 6 women judged slip was light and smooth
Poor: 0 to 2 women judged slip was light and smooth

Usability (Affinity With Skin)

A test of the feeling in use was performed by an expert panel of women (10). The affinity with the skin was evaluated by them by the following evaluation criteria:

Evaluation Criteria

Very good: All 10 women judged affinity with the skin was good
Good: 7 to 9 women judged affinity with the skin was good
Fair: 3 to 6 women judged affinity with the skin was good
Poor: 0 to 2 women judged affinity with the skin was good

Usability (Stickiness)

A test of the feeling in use was performed by an expert panel consisting of women (10). The stickiness was evaluated by them by the following evaluation criteria:

Evaluation Criteria

Very good: All 10 women judged feeling was moist with no stickiness

Good: 7 to 9 women judged feeling was moist with no stickiness

Fair: 3 to 6 women judged feeling was moist with no stickiness

Poor: 0 to 2 women judged feeling was moist with no stickiness

Usability (Clean Feeling)

A test of the feeling in use was performed by an expert panel of women (10). The clean feeling was evaluated by them by the following evaluation criteria:

Evaluation Criteria

Very good: All 10 women judged feeling was clean
Good: 7 to 9 women judged feeling was clean
Fair: 3 to 6 women judged feeling was clean
Poor: 0 to 2 women judged feeling was clean

Usability (Emollient Sensation: Feeling of Elasticity in Skin)

A test of the feeling in use was performed by an expert panel consisting of women (10). The emollient sensation was evaluated by them by the following evaluation criteria:

Evaluation Criteria

Very good: All 10 women judged there was emollient sensation
Good: 7 to 9 women judged there was emollient sensation
Fair: 3 to 6 women judged there was emollient sensation
Poor: 0 to 2 women judged there was emollient sensation

Usability (Firm Feeling: Feeling of Firmness of Skin)

A test of the feeling in use was performed by an expert panel consisting of women (10). The firmness was evaluated by them by the following evaluation criteria:

Evaluation Criteria

Very good: All 10 women judged there was firmness
Good: 7 to 9 women judged there was firmness
Fair: 3 to 6 women judged there was firmness
Poor: 0 to 2 women judged there was firmness

Table I-3

| | Ex. 1-3 | Ex. 1-4 | Ex. 1-5 | Ex. 1-6 | Ex. I-7 | Ex. I-8 | Ex. 1-9 | Ex. 1-10 |
|---|---|---|---|---|---|---|---|---|
| (1) 2-ethylhexyl 2-ethylhexanoate [(a) ingredient] | 0.5 | - | 10.0 | - | 4.0 | 3.0 | 10.0 | 5.0 |
| (2) Isononyl 2-ethylhexanoate [(a) ingredient] | 0.5 | 1.0 | - | 20.0 | 4.0 | 5.0 | - | 5.0 |

(continued)

| | Ex. 1-3 | Ex. 1-4 | Ex. 1-5 | Ex. 1-6 | Ex. I-7 | Ex. I-8 | Ex. 1-9 | Ex. 1-10 |
|---|---|---|---|---|---|---|---|---|
| (3) Cetyl 2-ethylhexanoate | - | - | - | - | - | - | - | - |
| (4) Octyl palmitate | - | - | - | - | - | - | - | - |
| (5) Dimethyl polysiloxane (5mPa*s) | - | - | - | - | - | - | - | - |
| (6) Stearyl alcohol [(b) ingredient] | 0.25 | - | 1.0 | 0.5 | 4.0 | - | 1.0 | 0.5 |
| (7) Behenyl alcohol [(b) ingredient] | 0.25 | - | - | 1.5 | 4.0 | 0.5 | - | 0.5 |
| (8) Stearic acid [(b) ingredient] | - | 0.25 | 0.5 | 0.5 | - | 1.0 | 0.5 | 0.5 |
| (9) Behenic acid [(b) ingredient] | - | 0.25 | - | - | - | - | - | 0.5 |
| (10) Cetostearyl glucoside(*1) | - | - | - | - | - | 0.3 | - | - |
| (11) Glyceryl monostearate (self emulsifying)(*2) | - | - | - | - | - | - | - | - |
| (12) POE (5) glyceryl monooleate(*3)[(d) ingredient] | - | - | - | - | - | - | 1.0 | - |
| (13) POE (10) monostearate(*4)[(d) ingredient] | 0.5 | - | - | 1.5 | - | 2.5 | - | 1.0 |
| (14) POE (20) phytosterol(*5)[(d) ingredient] | - | - | - | - | 2.0 | - | 3.0 | - |
| (15) POE (21) stearyl ether(*6)[(d) ingredient] | - | 1.0 | 3.0 | 1.5 | - | - | 3.0 | 4.0 |
| (16) AMPS homopolymer(*7)[(c) ingredient] | 0.1 | - | 1.0 | 1.5 | 0.1 | 2.5 | 0.5 | 0.25 |
| (17) Amide acrylate/ AMPS copolymer(*6)[ (c) ingredient] | - | 0.5 | - | 1.0 | - | - | - | - |
| (18) Dimethylacrylamide/ AMPS cross polymer(*9)[(c) ingredient] | - | - | - | - | - | 2.5 | 0.5 | - |
| (19) Carboxyvinyl polymer | - | - | - | - | - | - | - | - |
| (20) Paraben | 0.15 | 0.15 | 0.15 | 0.15 | 0.15 | 0.15 | 0.15 | 0.15 |
| (21) Edetate | 0.05 | 0.05 | 0.05 | 0.05 | 0.05 | 0.05 | 0.05 | 0.05 |

(continued)

| | Ex. 1-3 | Ex. 1-4 | Ex. 1-5 | Ex. 1-6 | Ex. I-7 | Ex. I-8 | Ex. 1-9 | Ex. 1-10 |
|---|---|---|---|---|---|---|---|---|
| (22) 1,3-butylene glycol | 7.0 | 7.0 | 7.0 | 7.0 | 7.0 | 7.0 | 7.0 | 7.0 |
| (23) Sodium hydroxide | - | 0.02 | 0.02 | 0.02 | - | 0.03 | 0.02 | 0.01 |
| (24) Ion exchanged water | Bal. | Bal. | Bal. | Bal. | Bal. | Bal. | Bal. | Bal. |
| Stability (50°C, 1 month) | Good | Good | Good | Good | Good | Good | Good | Good |
| Useability (Slip on skin) | Very good | Very good | Very good | Very good | Good | Very good | Good | Very good |
| (Affinity with the skin) | Very good | Very good | Very good | Good | Good | Very good | Very good | Very good |
| (Stickiness) | Very good | Very good | Very good | Very good | Good | Very good | Good | Good |
| (Freshness) | Very good | Very good | Very good | Good | Good | Very good | Very good | Good |
| (Emollient feeling) | Good | Good | Very good | Very good | Very good | Very good | Very good | Very good |
| (Firm feeling) | Good | Very good | Very good | Very good | Very good | Very good | Good | Very good |

Table 1-4

| | Comp. Ex. I-1 | Comp. Ex. 1-2 | Comp. Ex. 1-3 | Comp. Ex. 1-4 | Comp. Ex. 1-5 | Comp. Ex. 1-6 | Comp. Ex. 1-7 | Comp. Ex. I-8 |
|---|---|---|---|---|---|---|---|---|
| (1) 2-ethylhexyl 2-ethylhexanoate [(a) ingredient] | - | - | 10.0 | - | 4.0 | - | 10.0 | - |
| (2) Isononyl 2-ethylhexanoate [(a) ingredient] | - | - | - | 20.0 | 4.0 | - | - | - |
| (3) Cetyl 2-ethylhexanoate | 0.5 | 2.5 | - | - | - | - | - | - |
| (4) Octyl palmitate | 0.5 | 2.5 | - | - | - | - | - | 5.0 |
| (5) Dimethyl polysiloxane (5mPa*s) | - | - | - | - | - | 10.0 | - | 5.0 |
| (6) Stearyl alcohol [(b) ingredient] | 0.25 | - | 1.0 | 0.5 | - | - | 1.0 | - |
| (7) Behenyl alcohol [(b) ingredient] | 0.25 | - | - | 1.5 | - | 0.5 | - | - |
| (8) Stearic acid [(b) ingredient] | - | 0.25 | 0.5 | 0.5 | - | 1.0 | 0.5 | - |
| (9) Behenic acid [(b) ingredient] | - | 0.25 | - | - | - | - | - | - |

(continued)

| | Comp. Ex. I-1 | Comp. Ex. 1-2 | Comp. Ex. 1-3 | Comp. Ex. 1-4 | Comp. Ex. 1-5 | Comp. Ex. 1-6 | Comp. Ex. 1-7 | Comp. Ex. I-8 |
|---|---|---|---|---|---|---|---|---|
| (10) Cetostearyl glucoside(*1) | - | - | - | 1.0 | - | 0.3 | - | - |
| (11) Glyceryl monostearate (self emulsifying)(*2) | - | - | 3.0 | 1.0 | - | - | - | - |
| (12) POE (5) glyceryl monooleate(*3)[(d) ingredient] | - | - | - | - | - | - | 1.0 | - |
| (13) PEG (10) monostearate (*4)[(d) ingredient] | 0.5 | - | - | - | 1.0 | 2.5 | - | 1.0 |
| (14) POE (20) phytosterol(*5)[(d) ingredient] | - | - | - | - | - | - | 3.0 | - |
| (15) POE (21) stearyl ether(*6)[(d) ingredient] | - | 1.0 | - | - | 1.0 | - | 3.0 | 4.0 |
| (16) AMPS homopolymer(*7)[(c) ingredient] | 0.1 | - | - | 2.5 | 0.1 | 2.5 | - | 0.25 |
| (17) Amide acrylate/ AMPS copolymer(*8)[(c) ingredient] | - | 0.5 | - | - | - | - | - | - |
| (18) Dimethylacrylamide/ AMPS cross polymer(*9)[(c) ingredient] | - | - | 1.0 | - | - | 2.5 | - | - |
| (19) Carboxyvinyl polymer | 0.1 | - | - | - | - | - | - | - |
| (20) Paraben | 0.15 | 0.15 | 0.15 | 0.15 | 0.15 | 0.15 | 0.15 | 0.15 |
| (21) Edetate | 0.05 | 0.05 | 0.05 | 0.05 | 0.05 | 0.05 | 0.05 | 0.05 |
| (22) 1,3-butylene glycol | 7.0 | 7.0 | 7.0 | 7.0 | 7.0 | 7.0 | 7.0 | 7.0 |
| (23) Sodium hydroxide | 0.01 | 0.02 | 0.02 | 0.02 | - | 0.03 | 0.02 | 0.01 |
| (24) Ion exchanged water | Bal. | Bal. | Bal. | Bal. | Bal. | Bal. | Bal. | Bal. |
| Stability (50°C, 1 month) | Good | Good | Poor | Good | Good | Good | Poor | Good |
| Usability (Slip on skin) | Fair | Good | Poor | Fair | Good | Very good | Good | Fair |
| (Affinity with the skin) | Fair | Poor | Fair | Poor | Good | Poor | Very good | Poor |

(continued)

| | Comp. Ex. I-1 | Comp. Ex. 1-2 | Comp. Ex. 1-3 | Comp. Ex. 1-4 | Comp. Ex. 1-5 | Comp. Ex. 1-6 | Comp. Ex. 1-7 | Comp. Ex. I-8 |
|---|---|---|---|---|---|---|---|---|
| (Stickiness) | Good | Poor | Good | Fair | Very good | Good | Good | Very good |
| (Freshness) | Good | Fair | Poor | Poor | Good | Poor | Very good | Good |
| (Emollient feeling) | Good | Good | Poor | Very good | Poor | Fair | Very good | Fair |
| (Firm feeling) | Good | Good | Good | Fair | Poor | Good | Good | Poor |

Note that in Tables I-3 to I-4, the compounds shown below were used in the following products.
Cetostearyl glucoside(*1): "EMULGADE PL68/50" (HLB value 8.0, made by Cognis AG),
Glyceryl monostearate (self-emulsification type)(*2): "Nikkol MGS-ASEV" (HLB value 6.0, made by Nikko Chemicals Co. Ltd.),
POE (5) glyceryl monooleate(*3): "Nikkol TMGO-5" (HLB value 9.5, made by Nikko Chemicals Co. Ltd.),
PEG (10) monostearate(*4): "Emulex 810" (HLB value 11, made by Nihon-Emulsion Co., Ltd.),
POE (20) phytosterol(*5): "Nikkol BPS-20" (HLB value 15.5, made by Nikko Chemicals Co. Ltd.),
POE (21) stearyl ether (*6): "Brij721" (HLB value 15.5, made by Uniqema Ltd.),
AMPS homopolymer(*7): "Hostacerin AMPS" (made by CLARIANT,
Amide acrylate/AMPS copolymer(*8): "Sepigel 305" (made by SEPIC),
Dimethylacrylamide/AMPS cross polymer(*9): "SU polymer G-1" (made by Toho Chemical Industry Co. Ltd.)

It is understood from the results of Tables I-3 to I-4 that the skin creams of Examples I-3 to I-10 according to the present invention were superior in each of the effects of stability and useability (slip on skin, affinity with the skin, stickiness, clean feeling, emollient sensation and firm feeling). On the other hand, Comparative Examples I-1 to I-8 not satisfying the requirements of the present invention could not achieve the effects of both stability and useability.

The other examples of the present invention will now be shown.

Example I-11: Skin Cream

| | Formulated Ingredients | Mass% |
|---|---|---|
| (1) | Liquid paraffin | 2.0 |
| (2) | Decamethyl cyclopentasiloxane | 6.0 |
| (3) | Isononyl 2-ethylhexanoate | 6.0 |
| (4) | Glyceryl monostearate (self-emulsification type) ("EMALEX GMS-55FD", HLB value 7.0, made by Nihon-Emulsion Co., Ltd.) | 2.0 |
| (5) | Polyethylene glycol (30EO) monostearate ("EMALEX 830", HLB value 15, made by Nihon-Emulsion Co., Ltd.) | 4.0 |
| (6) | PEG (8) isostearate ("EMALEX PEIS-8EX", HLB | value |
| | 10.0, made by Nihon-Emulsion Co., Ltd.) | 1.0 |
| (7) | Cetyl alcohol | 2.5 |
| (8) | Batyl alcohol | 2.5 |
| (9) | Jojoba oil | 5.0 |
| (10) | Fragrance | 0.1 |
| (11) | Ion exchanged water | Bal. |
| (12) | 1,3-butylene glycol | 3.0 |
| (13) | Glucoside ascorbate | 2.0 |
| (14) | Paraben | 0.15 |
| (15) | Ethanol | 3.0 |
| (16) | Sodium hydroxide | 0.3 |

(continued)

| | Formulated Ingredients | Mass% |
|---|---|---|
| (17) | Vinyl pyrrolidone/AMPS copolymer ("ARISTOFLEX AVC"; made by Clariant Co. Ltd.) | 0.5 |
| (18) | Citric acid | 0.09 |
| (19) | Sodium citrate | 0.01 |

Method of Production

(1) to (10) were uniformly mixed and dissolved at 70°C (oil phase). On the other hand, (11) to (19) were uniformly mixed and dissolved at 70°C (aqueous phase). The oil phase was gradually added to the aqueous phase held at 70°C and the two were emulsified by a homo mixer. When the emulsification was ended, the result was rapidly cooled to 40°C or less to obtain the target skin cream.

Evaluation

The skin cream thus obtained was evaluated by the above-mentioned test and evaluation methods, whereupon it was superior in useability (useability evaluation: slip on skin, affinity with the skin, stickiness, clean feeling, emollient sensation, and firm feeling were all very good) and also superior in the stability (stability evaluation: good).

Example I-12: Oil-in-Water Emulsion Type Foundation

| | Formulated Ingredients | Mass% |
|---|---|---|
| (1) | Liquid lanolin | 2.0 |
| (2) | Hydrogenated polyisobutane ("PRISORINE 3758"; made by Uniqema Chemicals Ltd.) | 4.0 |
| (3) | 2-ethylhexyl 2-ethylhexanoate | 10.0 |
| (4) | Stearyl alcohol | 0.5 |
| (5) | Glyceryl monostearate ("NIKKOL MGS-F20V", value 7.0, made by Nikko Chemicals Co. Ltd.) | HLB 3.5 |
| (6) | Polyethylene glycol (40 EO) monostearate("NIKKOL MYS-40V", HLB value 17.5, made by Nikko Chemicals Co. Ltd.) | 0.5 |
| (7) | Fragrance | 0.1 |
| (8) | Ion exchanged water | Bal. |
| (9) | Dipropylene glycol | 2.5 |
| (10) | Ethanol | 1.0 |
| (11) | Paraben | 0.1 |
| (12) | Talc | 3.0 |
| (13) | Titanium dioxide | 5.0 |
| (14) | Red iron oxide | 0.5 |
| (15) | Yellow iron oxide | 1.4 |
| (16) | Black iron oxide | 0.1 |
| (17) | Hydroxyethyl acrylate/AMPS copolymer (active ingredient 37.5%) ("SIMULGEL NS"; SEPIC Co. Ltd.) | 0.5 (0.19) |

Method of Production

(1) to (7) were uniformly mixed and dissolved at 70°C (oil phase). On the other hand, (8) to (17) were uniformly mixed and dispersed at 70°C (aqueous phase). The oil phase was gradually added to the aqueous phase held at 70°C and the two were emulsified by a homo mixer. When the emulsification was ended, the resultant mixture was rapidly cooled to 40°C or less to obtain the target oil-in-water emulsion type foundation.

Evaluation

The oil-in-water emulsion type foundation thus obtained was evaluated by the above-mentioned test and evaluation methods, whereupon it was superior in useability (useability evaluation: slip on skin, affinity with the skin, stickiness, clean feeling, emollient sensation, and firm feeling all very good) and also superior in the stability (stability evaluation: good).

Example I-13: Oil-in-Water Emulsion Type Sunscreen

| | Formulated Ingredients | Mass% |
|---|---|---|
| (1) | Octyl para-methoxycinnamate | 6.0 |
| (2) | Glyceryl octyl dipara-methoxycinnamate | 2.0 |
| (3) | 4-tert-butyl-4'-methoxydibenzoylmethane | 2.0 |
| (4) | Pentaerythrityl tetra(octanate/paramethoxy-cinnamate) | 3.0 |
| (5) | 2-ethylhexyl 2-ethylhexanoate | 7.5 |
| (6) | Isononyl 2-ethylhexanoate | 7.5 |
| (7) | Dimethyl polysiloxane (20 mPa·s) | 3.0 |
| (8) | Petrolatum | 0.5 |
| (9) | Glyceryl monooleate ("NIKKOL MGO", HLB value made by Nikko Chemicals Co. Ltd.) | 2.5, 1.2 |
| (10) | Polyethylene (45EO) glycol monostearate ("NIKKOL MYS-45MV", HLB value 18.0, made by Nikko Chemicals Co. Ltd.) | 1.0 |
| (11) | Ion exchanged water | Bal. |
| (12) | Dipropylene glycol | 6.0 |
| (13) | Ethanol | 3.0 |
| (14) | Amide acrylate/AMPS copolymer (active ingredient 40%) ("Sepigel 305"; made by SEPIC CO. Ltd.) | 1.0 (0.4) |
| (15) | Fragrance | 0.1 |

Method of Production

(1) to (10) were uniformly mixed and dissolved at 70°C (oil phase). On the other hand, (11) to (15) were uniformly mixed and dispersed at 70°C (aqueous phase). The oil phase was gradually added to the aqueous phase held at 70°C and the two were emulsified by a homo mixer. When emulsification was ended, the resultant mixture was rapidly cooled to 40°C or less to obtain the target oil-in-water emulsion type sunscreen.

Evaluation

The obtained oil-in-water emulsion type sunscreen was evaluated by the above-mentioned test and evaluation methods, whereupon it was superior in the useability (useability evaluation: slip on skin, affinity with the skin, stickiness, clean feeling, emollient sensation, and firm feeling: very good) and also was superior in the stability (stability evaluation: good).

Example I-14: Skin Cream

| | Formulated Ingredients | Mass% |
|---|---|---|
| (1) | Dimethyl polysiloxane (6 mPa·s) | 2.0 |
| (2) | Decamethyl cyclopentasiloxane | 6.0 |
| (3) | 2-ethylhexyl 2-ethylhexanoate | 12.0 |
| (4) | Glyceryl monostearate ("EMALEX GMS-10SE", HLB value 6.0, made by Nihon-Emulsion Co., Ltd.) | 0.5 |
| (5) | Polyethylene (10EO) glycol stearate ("NIKKOL MYS-10V", HLB value 11.0, made by Nikko Chemicals Co. Ltd.) | 5.0 |
| (6) | Cetostearyl alcohol | 2.5 |
| (7) | Batyl alcohol | 2.5 |

(continued)

| | Formulated Ingredients | Mass% |
|---|---|---|
| (8) | Fragrance | 0.1 |
| (9) | Ion exchanged water | Bal. |
| (10) | 1,3-butylene glycol | 3.0 |
| (11) | Arbutin | 5.0 |
| (12) | Magnesium ascorbyl phosphate ester | 1.0 |
| (13) | Paraben | 0.15 |
| (14) | Ethanol | 3.0 |
| (15) | Sodium hydroxide | 0.4 |
| (16) | Dimethyl acrylamide/AMPS copolymer ("SIMULGEL EPG"; SEPIC Co. Ltd.) | 0.5 |
| (17) | Citric acid | 0.09 |
| (18) | Sodium citrate | 0.01 |

Method of Production

(1) to (8) were uniformly mixed and dissolved at 70°C (oil phase). On the other hand, (9) to (18) were uniformly mixed and dispersed at 70°C (aqueous phase). The oil phase was gradually added to the aqueous phase held at 70°C and the two were emulsified by a homo mixer. When emulsification was ended, the resultant mixture was rapidly cooled to 40°C or less to obtain the target skin cream.

Evaluation

The skin cream obtained above was evaluated by the above-mentioned test and evaluation methods, whereupon it was superior in the useability (useability evaluation: slip on skin, affinity with the skin, stickiness, clean feeling, emollient sensation, and firm feeling: very good) and also superior in the stability (stability evaluation: good).

Example I-15: Skin Cream

| | Formulated Ingredients | Mass% |
|---|---|---|
| (1) | Liquid paraffin | 1.0 |
| (2) | Decamethyl cyclopentasiloxane | 6.0 |
| (3) | Isononyl 2-ethylhexanoate | 6.0 |
| (4) | Glyceryl monostearate ("NIKKOL GS-BSEV", HLB value 8.0, made by Nikko Chemicals Co. Ltd.) | 1.0 |
| (5) | Polyethylene glycol stearate (100EO) ("Mirj59P", HLB value 18.8, made by Uniqema Chemicals Ltd.) | 2.0 |
| (6) | PEG (30) glyceryl triisostearate ("EMALEX GWIS-330", HLB value 10, made by Nihon-Emulsion Co., Ltd.) | 0.5 |
| (7) | Cetyl alcohol | 2.5 |
| (8) | Batyl alcohol | 2.5 |
| (9) | Fragrance | 0.1 |
| (10) | 1,3-butylene glycol | 3.0 |
| (11) | Ion exchanged water | Bal. |
| (12) | Trimethylglycine | 1.0 |
| (13) | Potassium 4-methoxysalicylate | 2.0 |
| (14) | Phenoxy ethanol | 0.15 |
| (15) | Sodium hydroxide | 0.4 |
| (16) | AMPS homopolymer ("Hostacerin AMPS"; made by Clariant International Ltd.) | 0.5 |
| (17) | Citric acid | 0.09 |
| (18) | Sodium citrate | 0.01 |

Method of Production

(1) to (9) were uniformly mixed and dissolved at 70°C (oil phase). On the other hand, (10) to (18) were uniformly mixed and dissolved at 70°C (aqueous phase). The oil phase was gradually added to the aqueous phase held at 70°C and the two were emulsified by a homo mixer. When emulsification was ended, it was rapidly cooled to 40°C or less to obtain the target skin cream.

Evaluation

The skin cream obtained above was evaluated by the above-mentioned test and evaluation methods, whereupon it was superior in the useability (useability evaluation: slip on skin, affinity with the skin, stickiness, clean feeling, emollient sensation, and firm feeling: very good) and also superior in the stability (stability evaluation: good).
Prior to the examples, the test methods and evaluation methods used by the present invention will be explained.

Examples II-1 to II-18 and Comparative Examples II-1 to II-9

The formulations shown in the following Tables II-1 to II-3 were used to prepare sunscreen cosmetic compositions. Note that Examples II-1 to II-9 (Table II-1) are oil-in-water emulsion types, and Examples II-10 to II-18 (Table II-2) are water-in-oil emulsion types. Further, in Table II-3, Comparative Examples II-1 and II-7 are oil-in-water emulsion types, while Comparative Examples II-2 to II-6, II-8, and II-9 are water-in-oil emulsion types.
The sunscreen cosmetic compositions (samples) of Examples II-1 to II-18 and Comparative Examples II-1 to II-9 were evaluated for their sunscreen effects, dispersibility immediately after preparation, and stability by a stress test by the following evaluation criteria (temperature stability and transport stability). Further, 20 monitors were asked to use each cosmetic composition were given a questionnaire regarding the five items of slip/comfort in application, rough feeling, residue, stickiness, and moist feeling. From the results, the useability was evaluated. The results are shown respectively in Tables II-1 to II-3.

Sunscreen Effect

The SPF value of each sample was measured according to the "SPF Measurement Criteria" (established in 2000) formulated by the Japan Cosmetic Industry Association.

Evaluation Criteria

Very good: SPF value 50 or more and/or PA+++
Good: SPF value 30 to less than 50 and/or PA++
Fair: SPF value 20 to less than 30 and/or PA+
Poor: SPF value less than 20

Dispersibility Immediately After Production

After preparing each sample, part of the preparation was put on a glass slide and observed for dispersion of the powder by the naked eye and under a microscope.

Evaluation Criteria

Very good: Good. Aggregates of powder could not be observed
Good: Somewhat good. Aggregates of powder of less than 1 mm could be observed
Fair: Somewhat poor. Aggregates of powder of 1 to 2 mm could be observed
Poor: Poor. Aggregates of powder of 2 mm or more could be observed

Stability by Stress Test

(1) Temperature Stability

Each sample was filled into a glass bottle and a plastic container and was held under temperature conditions of 0°C, 25°C, 40°C, and 50°C for one month. Each sample was then investigated for the degree of change (presence of discoloration, odor, settling and aggregation of the powder, specific gravity, viscosity, and emulsion state) and the redispersibility of the powder after shaking (number of times of shaking necessary for dispersion, dispersibility, specific gravity, and viscosity).

(2) Rolling Stability

Each sample was filled in a screw tube and subjected to a 5 hour rolling test by a rolling tester at a speed of 30 rpm. Each sample was then investigated for the degree of change (presence of discoloration, odor, settling and aggregation of the powder, specific gravity, viscosity, and emulsion state) and the redispersibility of the powder after rolling testing (number of times of shaking necessary for dispersion, dispersibility, specific gravity, and viscosity).

Evaluation Criteria

(i) Degree of Change of the Formulation

Very good: Good. Almost the same condition as production Good: Somewhat good. Discoloration and change in odor, settling and aggregation of powder, change in specific gravity, change in viscosity, and separation of sample could be somewhat observed
Fair: Somewhat poor. Discoloration and change in odor, settling and aggregation of powder, change in specific gravity, change in viscosity, and separation of sample could be clearly observed
Poor: Poor. Discoloration and change in odor, settling and aggregation of the powder, change in specific gravity, change in viscosity, and separation of sample could be remarkably noticed.

(ii) Redispersibility of Powder after Shaking

Very good: Good. Rapidly recovers to substantially same state as at production
Good: Somewhat good. Number of times of shaking is required, but recovers to state close to at production Fair: Somewhat poor. If increasing times of shaking, recovers, but is poor compared to state at production Poor: Poor. Regardless of the number of times of shaking, recovery was not recognized and redispersibility of powder was remarkably poor

Feeling in Use

The above 5 times (slip/comfort in application, rough feeling, residue, stickiness, and moist feeling) were evaluated by the following criteria from the results of a questionnaire.

Evaluation Criteria

Very good: 15 or more out of 20 answered it was good
Good: 10 to 14 out of 20 answered it was good
Fair: 5 to 9 out of 20 answered it was good
Poor: less than 4 out of 20 people answered it was good

Table II-1

| | Ex. II-1 | Ex. II-2 | Ex. II-3 | Ex. II-4 | Ex. II-5 | Ex. II-6 | Ex. II-7 | Ex. II-8 | Ex. II-9 |
|---|---|---|---|---|---|---|---|---|---|
| (1) Isononyl 2-ethylhexanoate [(a) ingredient] | 1.0 | 20.0 | 20.0 | 15.0 | 15.0 | 15.0 | 10.0 | 10.0 | 10.0 |
| (2) 2-ethylhexyl 2-ethylhexanoate [(a) ingredient] | - | - | 20.0 | 15.0 | 15.0 | 15.0 | 10.0 | 10.0 | 10.0 |
| (3) Isononyl isononate | - | - | - | - | - | - | - | - | - |
| (4) Cetyl octanoate | - | - | - | - | - | - | - | - | - |
| (5) 2-ethylhexyl paramethoxycinnamate [(b) ingredient] | 7.5 | 7.5 | 7.5 | 1.5 | 5.0 | 10.0 | 1.5 | 1.5 | 1.5 |
| (6) Octocrylene [(b) ingredient] | 5.0 | 5.0 | 5.0 | 1.5 | 5.0 | 10.0 | 1.5 | 1.5 | 1.5 |
| (7) Zinc oxide [(c) ingredient] | 10.0 | 10.0 | 10.0 | 10.0 | 10.0 | - | 9.0 | 37.0 | - |
| (8) Hydrophobic treated zinc oxide(*1)[(c) ingredient] | - | - | - | - | - | - | - | | - |
| (9) Titanium dioxide [(c) ingredient] | 1.0 | 1.0 | - | 1.0 | 1.0 | 1.0 | 1.0 | 3.0 | 5.0 |
| (10) Hydrophobic treated titanium dioxide(*2)[(c) ingredient] | - | - | - | - | - | - | - | - | - |
| (11) Polyoxyethylene methyl polysiloxane copolymer(*3)[(e) ingredient] | 3.0 | - | 3.0 | 3.0 | 3.0 | 3.0 | - | - | 3.0 |
| (12) Glyceryl monostearate(*4)[(e) ingredient] | - | 3.0 | - | - | - | - | 3.0 | 3.0 | - |
| (13) Alkyl chain·silicone chain branched polyoxyethylene methyl polysiloxane copolymer(*5)[(e) ingredient] | - | - | - | - | - | - | - | - | - |
| (14) Polyglyceryl-2 diisostearate(*6)[(e) ingredient] | - | - | - | - | - | - | - | - | - |
| (15) Dimethyl polysiloxane (6mPa·s)[(d) ingredient](*7) | 15.0 | 15.0 | 5.0 | 15.0 | 15.0 | 15.0 | 15.0 | 15.0 | 15.0 |
| (16) Trimethyl siloxysilicate·decamethyl cyclotetrasiloxane mixture | 1.0 | 1.0 | 1.0 | 1.0 | 1.0 | 1.0 | 1.0 | 1.0 | 1.0 |
| (17) Purified water | Bal. | Bal. | Bal. | Bal. | Bal. | Bal. | Bal. | Bal. | Bal. |
| (18) Ethanol | - | 3.0 | 3.0 | 3.0 | 3.0 | 3.0 | 3.0 | 3.0 | 3.0 |
| (19) Paraben | 0.1 | 0.1 | 0.1 | 0.1 | 0.1 | 0.1 | 0.1 | 0.1 | 0.1 |
| (20) Fragrance | 0.1 | 0.1 | 0.1 | 0.1 | 0.1 | 0.1 | 0.1 | 0.1 | 0.1 |
| Sunscreen effect | Good | Good | Good | Good | Good | Good | Good | Good | Good |
| Dispersibility immediately after production | Good | V. good | V. good | V. good | V. good | V. good | V. good | Good | V.good |

(continued)

| | | | Ex. II-1 | Ex. II-2 | Ex. II-3 | Ex. II-4 | Ex. II-5 | Ex. II-6 | Ex. II-7 | Ex. II-8 | Ex. II-9 |
|---|---|---|---|---|---|---|---|---|---|---|---|
| Stability by stress test | Temperature stability | Degree of change | Good | V.good | V.good | V.good | V.good | V.good | V.good | Good | V.good |
| | | Redispersibility of powder | Good | V.good | V.good | V.good | V.good | V.good | V.good | Good | V.good |
| | Rolling stability | Degree of change | Good | V.good | V.good | V. good | V.good | V. good | V. good | Good | V.good |
| | | Redispersibility of powder | Good | V.good | V.good | V.good | V.good | V.good | V.good | Good | V.good |
| Useability | | Slip/comfort in application | V.good | V.good | V.good | V.good | V.good | V.good | V.good | Good | V.good |
| | | Roughness | V.good | Good | V.good | V.good | V.good | V.good | Good | Good | V.good |
| | | Residue | V.good | Good | V.good | V.good | V.good | V.good | Good | Good | Good |
| | | Stickiness | V.good | Good | Good | Good | V.good | V.good | Good | V.good | V.good |
| | | Moisture | Good | Good | V. good | V. good | V. good | V. good | Good | Good | V. good |

Table II-2

| | Ex. II-10 | Ex. II-11 | Ex. II-12 | Ex. II-13 | Ex. II-14 | Ex. II-15 | Ex. II-16 | Ex. II-17 | Ex. II-18 |
|---|---|---|---|---|---|---|---|---|---|
| (1) Isononyl 2-ethylhexanoate [(a) ingredient] | 1.0 | 20.0 | 20.0 | 15.0 | 15.0 | 15.0 | 10.0 | 10.0 | 10.0 |
| (2) 2-ethylhexyl 2-ethylhexanoate [(a) ingredient] | - | - | 20.0 | 15.0 | 15.0 | 15.0 | 10.0 | 10.0 | 10.0 |
| (3) Isononyl isononate | - | - | - | - | - | - | - | - | - |
| (4) Cetyl octanoate | - | - | - | - | - | - | - | - | - |
| (5) 2-ethylhexyl paramethoxycinnamate [(b) ingredient] | 7.5 | 7.5 | 7.5 | 1.5 | 5.0 | 10.0 | 1.5 | 1.5 | 1.5 |
| (6) Octocrylene [(b) ingredient] | 5.0 | 5.0 | 5.0 | 1.5 | 5.0 | 10.0 | 1.5 | 1.5 | 1.5 |
| (7) Zinc oxide [(c) ingredient] | - | - | - | - | - | - | - | - | - |
| (8) Hydrophobic treated zinc oxide[*1] [(c) ingredient] | 10.0 | 10.0 | 10.0 | 10.0 | 10.0 | 10.0 | 9.0 | 20.0 | 35.0 |
| (9) Titanium dioxide [(c) ingredient] | - | - | - | - | - | - | - | - | - |
| (10) Hydrophobic treated titanium dioxide[*2] [(c) ingredient] | 1.0 | 1.0 | 1.0 | 1.0 | 1.0 | 1.0 | 1.0 | 3.0 | 5.0 |
| (11) Polyoxyethylene methyl polysiloxane copolymer[*3] [(e) ingredient] | - | - | - | - | - | - | - | - | - |
| (12) Glyceryl monostearate[*4] [(e) ingredient] | - | - | - | - | - | - | - | - | - |
| (13) Alkyl chain·silicone chain branched polyoxyethylene methyl polysiloxane copolymer[*5] [(e) ingredient] | 2.0 | 2.0 | 3.0 | 3.0 | 3.0 | 3.0 | 3.0 | 3.0 | 3.0 |
| (14) Polyglyceryl-2 diisostearate[*6] [(e) ingredient] | - | - | - | - | - | - | - | - | - |
| (15) Dimethyl polysiloxane (6mPa·s) [(d) ingredient][*7] | 15.0 | 15.0 | 5.0 | 15.0 | 15.0 | 15.0 | 15.0 | 15.0 | 15.0 |
| (16) Trimethyl siloxysilicate·decamethyl cyclotetrasiloxane mixture | 1.0 | 1.0 | 1.0 | 1.0 | 1.0 | 1.0 | 1.0 | 1.0 | 1.0 |
| (17) Purified water | Bal. | Bal. | Bal. | Bal. | Bal. | Bal. | Bal. | Bal. | Bal. |
| (18) Ethanol | - | 3.0 | 3.0 | 3.0 | - | 3.0 | 3.0 | - | 3.0 |
| (19) Paraben | 0.1 | 0.1 | 0.1 | 0.1 | 0.1 | 0.1 | 0.1 | 0.1 | 0.1 |
| (20) Fragrance | 0.1 | 0.1 | 0.1 | 0.1 | 0.1 | 0.1 | 0.1 | 0.1 | 0.1 |
| Sunscreen effect | Good | V. good | V. good | Good | V.good | Good | Good | Good | Good |
| Dispersibility immediately after production | Good | V.good | V.good | V.good | V.good | V.good | V.good | Good | V.good |

(continued)

| | | | Ex. II-10 | Ex. II-11 | Ex. II-12 | Ex. II-13 | Ex. II-14 | Ex. II-15 | Ex. II-16 | Ex. II-17 | Ex. II-18 |
|---|---|---|---|---|---|---|---|---|---|---|---|
| Stability by stress test | Temperature stability | Degree of change | Good | V.good | V.good | V.good | V.good | V.good | V.good | Good | V.good |
| | | Redispersibility of powder | Good | V.good | V.good | V.good | V.good | V.good | V.good | Good | V.good |
| | Rolling stability | Degree of change | Good | V.good | V.good | V.good | V.good | V.good | V.good | Good | V.good |
| | | Redispersibility of powder | Good | V.good | V.good | V.good | V.good | V.good | V.good | Good | V.good |
| Useability | Slip/comfort in applicationeling | | V.good | V.good | V.good | V.good | V.good | V.good | V.good | Good | V.good |
| | Roughness | | V.good | V.good | V.good | V.good | V.good | V.good | V.good | Good | V.good |
| | Residue | | V.good | V.good | V.good | V.good | V.good | V.good | V.good | Good | Good |
| | Stickiness | | V.good | V.good | Good | Good | V.good | V.good | V.good | V.good | V.good |
| | Moisture | | Good | V.good | V.good | V.good | V.good | V.good | V.good | V.good | V.good |

Table II-3

| | C. Ex. II-1 | C. Ex. II-2 | C. Ex. II-3 | C. Ex. II-4 | C. Ex. II-5 | C. Ex. II-6 | C. Ex. II-7 | C. Ex. II-8 | C. Ex. II-9 |
|---|---|---|---|---|---|---|---|---|---|
| (1) Isononyl 2-ethylhexanoate [(a) ingredient] | - | - | - | 15.0 | 15.0 | 15.0 | - | - | 10.0 |
| (2) 2-ethylhexyl 2-ethylhexanoate [(a) ingredient] | - | - | - | 15.0 | 15.0 | 15.0 | - | - | 10.0 |
| (3) Isononyl isononate | 10.0 | 20.0 | - | - | - | - | - | - | - |
| (4) Cetyl octanoate | - | - | 20.0 | - | - | - | 20.0 | - | - |
| (5) 2-ethylhexyl paramethoxycinnamate [(b) ingredient] | 7.5 | 7.5 | 7.5 | - | 7.5 | 7.5 | 1.5 | 1.5 | 1.5 |
| (6) Octocrylene [(b) ingredient] | 5.0 | 5.0 | 5.0 | - | 5.0 | 5.0 | 1.5 | 1.5 | 1.5 |
| (7) Zinc oxide [(c) ingredient] | 1.0 | - | - | - | - | - | 9.0 | - | - |
| (8) Hydrophobic treated zinc oxide(*1) [(c) ingredient] | - | 10.0 | 10.0 | 10.0 | 10.0 | 5.0 | - | 20.0 | 10.0 |
| (9) Titanium dioxide [(c) ingredient] | 10.0 | 1.0 | - | - | - | - | 1.0 | - | - |
| (10) Hydrophobic treated titanium dioxide(*2) [(c) ingredient] | - | - | 1.0 | 1.0 | 1.0 | 5.0 | - | 3.0 | 1.0 |
| (11) Polyoxyethylene methyl polysiloxane copolymer(*3)[(e) ingredient] | - | 3.0 | - | - | - | - | 3.0 | - | - |
| (12) Glyceryl monostearate(*4) [(e) ingredient] | 3.0 | - | - | - | - | - | - | - | - |
| (13) Alkyl chain·silicone chain branched polyoxyethylene methyl polysiloxane copolymer(*5) [(e) ingredient] | - | 0.5 | - | 3.0 | - | 3.0 | - | 3.0 | 3.0 |
| (14) Polyglyceryl-2 diisostearate(*6) [(e) ingredient] | - | - | 3.0 | - | 3.0 | - | - | - | - |
| (15) Dimethyl polysiloxane (6mPa·s) [(d) ingredient](*7) | 15.0 | 15.0 | 5.0 | 15.0 | - | 15.0 | 15.0 | 30.0 | 15.0 |
| (16) Trimethyl siloxysilicate·decamethyl cyclotetrasiloxane mixture | 1.0 | 1.0 | 1.0 | 1.0 | 1.0 | 1.0 | 1.0 | 1.0 | 1.0 |
| (17) Purified water | Bal. | Bal. | Bal. | Bal. | Bal. | Bal. | Bal. | Bal. | Bal. |
| (18) Ethanol | - | 3.0 | 3.0 | 3.0 | - | 3.0 | 3.0 | - | 3.0 |
| (19) Paraben | 0.1 | 0.1 | 0.1 | 0.1 | 0.1 | 0.1 | 0.1 | 0.1 | 0.1 |

(continued)

| | | | C. Ex. II-1 | C. Ex. II-2 | C. Ex. II-3 | C. Ex. II-4 | C. Ex. II-5 | C. Ex. II-6 | C. Ex. II-7 | C. Ex. II-8 | C. Ex. II-9 |
|---|---|---|---|---|---|---|---|---|---|---|---|
| (20) Fragrance | | | 0.1 | 0.1 | 0.1 | 0.1 | 0.1 | 0.1 | 0.1 | 0.1 | 0.1 |
| Sunscreen effect | | | Good | V.good | V.good | Fair | Fair | Poor | Good | Poor | Poor |
| Dispersibility immediately after production | | | Good | V.good | Fair | V.good | V. good | V.good | Good | Poor | V.good |
| Stability bystress test | Temperature stability | Degree of change | Fair | Fair | Poor | Fair | V.good | V.good | Fair | Poor | V.good |
| | | Redispersibility of powder | Fair | Fair | Fair | Fair | V.good | V.good | Poor | Poor | V.good |
| | Rolling stability | Degree of change | Poor | Poor | Poor | Good | V.good | V.good | Poor | Poor | V.good |
| | | Redispersibility of powder | Poor | Fair | Poor | Fair | V.good | V.good | Poor | Poor | V.good |
| Usability | Slip/comfort in application | | Good | Good | Fair | Good | Poor | V.good | Poor | Poor | V.good |
| | Roughness | | V.good | Good | Fair | V.good | Fair | V.good | Fair | Poor | V.good |
| | Residue | | V.good | V.good | Good | V.good | Good | V.good | Fair | Poor | Good |
| | Stickiness | | Fair | Fair | Poor | Good | Good | V.good | Poor | Good | V.good |
| | Moisture | | Good | V.good | V.good | Good | Poor | V.good | V.good | Good | V.good |

Note that, in Tables II-1 to II-3, the compounds shown below used the following products.
Hydrophobic treated zinc oxide(*1): Methyl hydrogen polysiloxane treated zinc oxide ("SS-ActivoxC80"; made by Showa Denko K.K.),
Hydrophobic treated titanium dioxide(*2): Aluminum stearate treated titanium dioxide ("MT-014"; made by Tayca Corporation),
Polyoxyethylene methyl polysiloxane copolymer(*3): PEG-11 methyl ether dimethicone ("KF-6011", HLB value =14.5; made by Shin-Etsu Chemical Co. Ltd.),
Glyceryl monostearate(*4): "Nikkol MGS-ASEV", HLB value=6.0 (made by Nikko Chemicals Co. Ltd.),
Alkyl chain-silicone chain branched type polyoxyethylene methyl polysiloxane copolymer(*5): Lauryl PEG-9 polydimethylsiloxyethyl dimethicone ("KF-6038", HLB value=3.0; made by Shin-Etsu Chemical Co. Ltd.),
Polyglyceryl-2 diisostearate(*6): "Wogel 18DV" (made by Matsumoto Trading Co. Ltd.), and
Dimethyl polysiloxane (*7):"SH200C-5cs" (made by Dow Corning Toray Silicones Co. Ltd.)

As is clear from the results of Tables II-1 to II-3, the oil-in-water or the water-in-oil type emulsion sunscreen cosmetic compositions of the present invention containing the ingredients (a) to (d) are significantly superior in the dispersibility and stability compared to the cosmetics of the Comparative Examples and along with this are superior in the sunscreen effect and the useability.

The cosmetic compositions of the present invention had good slip on the skin, had no grinding or rough feeling during use, furthermore, had a smooth feeling, had no stickiness, and was superior in feeling during use. Furthermore, they left no residue on the skin and were superior even in cosmetic finish.

These effects, as will be understood from the Comparative Examples, are distinctive effects obtained by using specific 2-ethylhexanoate esters as ester oils.

The other examples of the present invention are now shown.

Example II-19: Oil-in-Water Emulsion Type Sunscreen Cosmetic Composition (Cream Type)

| | Formulated Ingredients | Mass% |
|---|---|---|
| | (Aqueous phase part) | |
| (1) | 1,3-butylene glycol | 7.0 |
| (2) | Zinc oxide ("Nanofine-K-LP"; made by Sakai Chemical Industry Co. Ltd.) | 5.0 |
| (3) | Disodium edetate | 0.05 |
| (4) | Triethanolamine | 1.0 |
| (5) | 2,2'-methylene bis[6-(2H-benzotriazol-2-yl)-(1,1,3,3,-tetramethylbutyl)phenol] ("TinosorbM; made by Ciba Speciality Chemicals Ltd.) | 1.0 |
| (6) | Ion exchanged water (Oil phase part) | Bal. |
| (7) | 4-methyl benzylidene camphor ("Neo Heliopan (NEO HELIOPAN) MBC"; made by Symrise AG) | 2.0 |
| (8) | Octyl salicylate ("Neo Heliopan OS"; made by Symrise AG) | 4.0 |
| (9) | Isononyl 2-ethylhexanoate | 10.0 |
| (10) | Decamethyl cyclopentasiloxane | 10.0 |
| (11) | Petrolatum | 5.0 |
| (12) | Stearyl alcohol | 3.0 |
| (13) | Stearic acid | 3.0 |
| (14) | Glyceryl monostearate | 3.0 |
| (15) | Ethyl polyacrylate | 1.0 |
| (16) | Glutathione | q.s. |
| (17) | Paraben | q.s. |
| (18) | Fragrance | q.s. |

Method of Production

The oil phase part and aqueous phase part were respectively heated to 70°C to be dissolved. The aqueous phase part was agitated to sufficiently disperse the titanium dioxide, the oil phase part was added, and the resultant mixture was emulsified using a homogenizer. The emulsion was cooled using a heat exchanger.

The oil-in-water emulsion type sunscreen cosmetic composition obtained above had no grinding and rough feeling during use, furthermore had a smooth feeling, had no stickiness, and was superior in feeling during use. Furthermore, it left no residue on the skin and was superior even in cosmetic finish. Further, an SPF value 22 and a value of PA+ were shown by the above-mentioned SPF measurement.

Example II-20: Oil-in-Water Emulsion Type Sunscreen cosmetic composition (Milky Liquid Type)

| | Formulated Ingredients | Mass% |
|---|---|---|
| | (Aqueous phase part) | |
| (1) | Dipropylene glycol | 6.0 |

(continued)

| | Formulated Ingredients | Mass% |
|---|---|---|
| (2) | Ethanol | 3.0 |
| (3) | Hydroxyethylcellulose | 0.3 |
| (4) | Phenylbenzimidazole sulfonate ("NEO HELIOPAN HYDRO"; made by Symrise AG) | 1.5 |
| (5) | Benzylidene camphor sulfonic acid ("Mexoryl (Mexoryl) SL"; made by Chimex Inc. Ltd.) | 1.5 |
| (6) | Zinc oxide ("Zinc Oxide Neutral", particle size 40 nm, made by Symrise AG) | 3.0 |
| (7) | Titanium dioxide ("STR-100C"; made by Sakai Chemical Industry Co. Ltd.) | 3.0 |
| (8) | Ion exchanged water (Oil phase) | Bal. |
| (9) | Isononyl 2-ethylhexanoate | 4.0 |
| (10) | 2,4-bis{[4-(2-ethylhexyloxy)-2-hydroxy]phenyl}-6-(4-methoxyphenyl)-1,3,5-triazine ("Tinosorb S"; made by Ciba Speciality Chemicals Inc.) | 0.5 |
| (11) | Ethylhexyl methoxycinnamate ("Parsol MCX"; made by Hoffmann-La Roche Ltd.) | 2.0 |
| (12) | 4-tert-butyl-4'-methoxybenzoyl methane ("Palsol 1789"; made by Hoffmann-La-Roche Ltd.) | 1.0 |
| (13) | Benzophenone-3 ("Uvinul M40"; made by BASF) | 1.0 |
| (14) | Oleyloleate | 5.0 |
| (15) | Dimethyl polysiloxane (6 mPa·s) ("KF-96A-6"; made by Shin-Etsu Chemical Co. Ltd.) | 3.0 |
| (16) | Petrolatum | 0.5 |
| (17) | Cetyl alcohol | 1.0 |
| (18) | Sorbitan sesquioleate ester | 0.8 |
| (19) | Tocopherol | q.s. |
| (20) | Fragrance | q.s. |

Method of Production

The oil phase part and aqueous phase part were respectively heated to 70°C to dissolve. The aqueous phase part was agitated to sufficiently disperse the titanium dioxide, the oil phase part was added, and the resultant mixture was emulsified using a homogenizer. The emulsion was cooled using a heat exchanger.

The oil-in-water emulsion type sunscreen cosmetic composition obtained had no grinding and rough feeling during use, had a smooth texture, had no stickiness, and was superior in feeling during use. Furthermore, it left no residue on the skin and was superior even in cosmetic finish. Further, an SPF value of 25 and a value of PA++ were shown by the above-mentioned SPF measurement.

Example II-21: Water-in-Oil Emulsion Type Sunscreen Cosmetic Composition (Cream Type)

| | Formulated Ingredients | Mass% |
|---|---|---|
| | (Aqueous phase part) | |
| (1) | 1,3-butylene glycol | 5.0 |
| (2) | Ion exchanged water (Oil phase) | Bal. |
| (3) | Isononyl 2-ethylhexanoate | 15.0 |
| (4) | Octocrylene ("NEO HELIOPAN 303"; made by Symrise AG) | 5.0 |
| (5) | Butylmethoxydibenzoyl methane ("NEO HELIOPAN"; made by Symrise AG) | 3.0 |
| (6) | 3-(4'-methyl benzylidene)-dl-camphor ("Eusolex 6300"; made by Merck & Co. Inc.) | 3.0 |
| (7) | Hydrophobic treated (alumina and fatty acid soap treated) titanium dioxide ("TTO-S-4"; made by Ishihara Sangyou Kaisha Ltd.) | 3.0 |
| (8) | Glyceryl diisostearate | 3.0 |
| (9) | Organic modified montmorillonite | 1.5 |
| (10) | Decamethyl cyclopentasiloxane | 10.0 |
| (11) | Paraben | q.s. |
| (12) | Fragrance | q.s. |

Method of Production

The oil phase part and aqueous phase part were respectively heated to 70°C to dissolve. The oil phase part was agitated to sufficiently disperse the titanium dioxide, then the aqueous phase part was added while homogenizing the two.

The water-in-oil emulsion type sunscreen cosmetic composition obtained had no grinding and rough feeling during use, had a smooth texture, had no stickiness, and was superior in feeling during use. Furthermore, it left no residue on the skin and was superior even in cosmetic finish. Further, an SPF value of 40 and a value of PA++ were shown by the above-mentioned SPF measurement.

Example II-22: Water-in-Oil Emulsion Type Sunscreen Cosmetic (Cream Type)

| | Formulated Ingredients | Mass% |
|---|---|---|
| | (Water phase part) | |
| (1) | 1,3-butylene glycol | 2.0 |
| (2) | Glycerin | 2.0 |
| (2) | Ion exchanged water (Oil phase) | Bal. |
| (3) | 2-ethylhexyl 2-ethylhexanoate | 13.0 |
| (4) | 2-ethylhexylisononyl | 10.0 |
| (5) | Isoamyl-p-methoxycinnamate ("NEO HELIOPAN E1000"; made by Symrise AG) | 2.0 |
| (6) | 2,4-bis-{[4-(2-ethylhexyloxy)-2-hydroxy]phenyl}-6-(4-methoxyphenyl)-1,3,5-triazine ("Tinosorb S"; made by Ciba Specialty Chemicals Inc.) | 3.0 |
| (7) | 3-(4'-methyl benzylidene)-dl-camphor ("Eusolex 6300"; made by Merck & Co. Inc.) | 3.0 |
| (8) | Hydrophobic treated (fatty acid soap treated) titanium dioxide ("MT-014"; made by Tayca Corporation) | 3.0 |
| (9) | PEG-9 polydimethylsiloxyethyl dimethicone ("KF-6028"; made by Shin-Etsu Chemical Co. Ltd.) | 3.0 |
| (10) | Dimethicone-(dimethicone/polyglycerin-3) cross polymer ("KSG-710"; made by Shin-Etsu Chemical Co. Ltd.) | 1.5 |
| (11) | Decamethyl cyclopentasiloxane | 5.0 |
| (12) | Dimethyl polysiloxane | 5.0 |
| (13) | Paraben | q.s. |
| (14) | Fragrance | q.s. |

Method of Production

The oil phase part and water phase part were respectively heated to 70°C to dissolve. The oil phase part was agitated to sufficiently disperse the titanium dioxide, then the aqueous phase part was added while homogenizing the two.

Method of Production

The oil phase part and aqueous phase part were respectively heated to 70°C to be dissolved. The aqueous phase part was agitated to sufficiently disperse the titanium dioxide, the oil phase part was added, and the resultant mixture was emulsified using a homogenizer. The emulsion was cooled using a heat exchanger.

The water-in-oil emulsion type sunscreen cosmetic composition obtained had no grinding and rough feeling during use, had a smooth texture, had no stickiness, and was superior in feeling during use. Furthermore, it left no residue on the skin and was superior even in cosmetic finish. Further, an SPF value of 50 and a value of PA+++ were shown by the above-mentioned SPF measurement.

Example II-23: Water-in-Oil Emulsion Type Sunscreen Cosmetic Composition (Milky Lotion Type)

| | Formulated Ingredients | Mass% |
|---|---|---|
| | (Aqueous phase part) | |
| (1) | 1,3-butylene glycol | 5.0 |
| (2) | Dipropylene glycol | 2.0 |
| (3) | Ion exchanged water (Oil phase) | Bal. |
| (4) | 2-ethylhexyl 2-ethylhexanoate | 2.0 |
| (5) | 2-ethylhexylisononyl | 2.0 |
| (6) | Etocrylene ("Uvinul N35"; made by BASF) | 5.0 |
| (7) | Ethylhexyl methoxycinnamate ("NEO HELIOPAN AV"; made by Symrise AG) | 5.0 |
| (8) | 1-(4-tert-butylphenyl)-3-(4-methoxyphenyl)propane-1,3-dion ("Eusolex 9020"; made by Merck & Co. Inc.) | 5.0 |
| (9) | Hydrophobic treated (dimethicone treated) zinc oxide ("MT-FINEX-50LP"; made by Sakai Chemical Industry Co. Ltd.) | 5.0 |
| (10) | Hydrophobic treated (alumina and dimethicone treated) titanium dioxide ("SAS-015"; made by Miyoshi-Kasei Industry Co. Ltd.) | 2.0 |
| (11) | PEG-3 dimethicone ("KF-6015"; made by Shin-Etsu Chemical Co. Ltd.) | 1.0 |
| (12) | PEG-11 methylether dimethicone ("KF-6018"; made by Shin-Etsu Chemical Co. Ltd.) | 2.5 |
| (13) | Decamethyl cyclopentasiloxane | 2.0 |
| (14) | Dimethyl polysiloxane | 2.0 |
| (15) | Paraben | q.s. |
| (16) | Fragrance | q.s. |

Method of Production

The oil phase part and aqueous phase part were respectively heated to 70°C to be dissolved. The oil phase part was agitated to sufficiently disperse the titanium dioxide, then the aqueous phase part was added while homogenizing the two.

Method of Production

The oil phase part and aqueous phase part were respectively heated to 70°C to dissolve. The aqueous phase part was agitated to sufficiently disperse the titanium dioxide, the oil phase part was added, and the resultant mixture was emulsified using a homogenizer. The emulsion was cooled using a heat exchanger.

The water-in-oil emulsion type sunscreen cosmetic composition obtained above had no grinding and rough feeling during use, had a smooth texture, had no stickiness, and was superior in feeling during use. Furthermore, it left no residue on the skin and was superior even in cosmetic finish. Further, an SPF value of 40 and a value of PA++ were shown by the above-mentioned SPF measurement.

## Claims

**1.** An external preparation for the skin comprising isononyl 2-ethylhexanoate and/or 2-ethylhexyl 2-ethylhexanoate.

**2.** An external preparation for the skin as claimed in claim 1, wherein the isononyl 2-ethylhexanoate and/or 2-ethylhexyl 2-ethylhexanoate are contained in an amount of 1.0 to 20.0 mass%.

**3.** An oil-in-water emulsion type skin cosmetic composition comprising (a) isononyl 2-ethylhexanoate and/or 2-ethylhexyl 2-ethylhexanoate, (b) one or more ingredients selected from higher fatty acids and higher alcohols, which are solid to semi-solid at an ordinary temperature (25°C), (c) a homopolymer, copolymer, cross polymer or a mixture thereof containing as a constituent unit one or more ingredients selected from 2-acrylamide-2-methylpropane sulfonic acid and acrylic acid and its derivatives and (d) one or more ingredients selected from nonionic surfactants having an HLB value of 9 or more.

**4.** An oil-in-water emulsion type skin cosmetic composition as claimed in claim 3, wherein the ingredient (a) is contained in an amount of 1.0 to 20.0 mass%, the ingredient (b) is contained in an amount of 0.5 to 8.0 mass%, the ingredient

(c) is contained in an amount of 0.1 to 5.0 mass% and the ingredient (d) is contained in an amount of 0.5 to 8.0 mass% in the total mass of the cosmetic composition.

**5.** An oil-in-water emulsion type skin cosmetic composition as claimed in claim 3 or 4, wherein the ingredient (c) is one or more ingredients selected from a vinyl pyrrolidone/AMPS copolymer, dimethylacrylamide/AMPS copolymer, acrylate amide/AMPS copolymer, cross polymer of dimethylacrylamide/AMPS cross-linked with methylene bisacrylamide, a mixture of polyacrylate amide and sodium polyacrylate, sodium acrylate/AMPS copolymer, hydroxyethyl acrylate/AMPS copolymer, ammonium polyacrylate, polyacrylamide/ammonium acrylate copolymer and acrylamide/sodium acrylate copolymer.

**6.** An oil-in-water emulsion type skin cosmetic composition as claimed in any one of claims 3 to 5, wherein the ingredient (d) is one or more ingredients selected from polyoxyethylene adducts and polyethyleneglycol adducts.

**7.** An oil-in-water emulsion type skin cosmetic composition as claimed in any one of claims 3 to 6, which uses, as the ingredient (d) a combination of a nonionic surfactant having an HLB value of 9 to less than 15 and a nonionic surfactant having an HLB value of 15 or more.

**8.** An oil-in-water or a water-in-oil emulsion type sunscreen cosmetic composition comprising (a) isononyl 2-ethylhexanoate and/or 2-ethylhexyl 2-ethylhexanoate, (b) an ultraviolet absorber, (c) an ultraviolet scatterer and (d) a silicone oil.

**9.** A sunscreen cosmetic composition as claimed in claim 8, wherein the ingredient (b) is one or more ingredients selected from para-aminobenzoic acid derivatives, salicylic acid derivatives, cinnamic acid derivatives, β,β-diphenyl acrylate derivatives, benzophenone derivatives benzylidene camphor derivatives, phenylbenzoimidazole derivatives, triazine derivatives, phenylbenzotriazole derivatives, anthranil derivatives, imidazoline derivatives, benzal malonate derivatives and 4,4-diarylbutadiene derivatives.

**10.** A sunscreen cosmetic composition as claimed in claim 8 or 9, wherein the ingredient (c) is zinc oxide and/or titanium dioxide.

**11.** A sunscreen cosmetic composition as claimed in claim 10, wherein the zinc oxide has an average primary particle size of 5 to 40 nm.

**12.** A sunscreen cosmetic composition as claimed in claim 10, wherein the titanium dioxide has an average primary particle size of 5 to 30 nm.

**13.** A sunscreen cosmetic composition as claimed in any one of claims 8 to 12, wherein the ingredient (a) is contained in an amount of 1 to 60 mass%, the ingredient (b) is contained in an amount of 3 to 20 mass%, the ingredient (c) is contained in an amount of 0.5 to 50 mass% and the ingredient (d) is contained in an amount of 1 to 70 mass% in the total mass of the cosmetic composition.

**14.** A sunscreen cosmetic composition as claimed in any one of claims 8 to 13, further containing (e) one or more ingredients selected from a silicone-based surfactant, glycerin or a polyglyceryl fatty acid ester.

**15.** A sunscreen cosmetic composition as claimed in claim 14, wherein the silicone-based surfactant as the ingredient (e) is one or more ingredients selected from poly(oxyethylene-oxypropylene) methyl polysiloxane copolymers, polyoxyethylene methyl polysiloxane copolymers, silicone chain branched type methyl polysiloxane copolymers, alkyl chain-silicone chain branched type polyoxyethylene methyl polysiloxane copolymers, cross-linkable polyoxyethylene methyl polysiloxane, cross-linked type polyoxyethylene methyl polysiloxane containing alkyl groups, branched type polyglycerin modified silicone, cross-linkable polyglycerin modified silicone, cross-linkable polyglycerin modified silicone containing alkyl groups and alkyl group branched type polyglycerin modified silicone.

**16.** A sunscreen cosmetic composition as claimed in claim 14 or 15, the ingredient (e) is contained in an amount of 0.01 to 20 mass% in the total mass of the cosmetic composition.

**INTERNATIONAL SEARCH REPORT**

International application No.
PCT/JP2006/321925

A. CLASSIFICATION OF SUBJECT MATTER
*A61K8/37*(2006.01)i, *A61K8/06*(2006.01)i, *A61Q17/04*(2006.01)i

According to International Patent Classification (IPC) or to both national classification and IPC

B. FIELDS SEARCHED

Minimum documentation searched (classification system followed by classification symbols)
A61K8/37, A61K8/06, A61Q17/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus/MEDLINE/BIOSIS/EMBASE/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | US 5711939 A (Dragoco Gerberding & Co.), 27 January, 1998 (27.01.98), Full text; particularly, Claim 1; column 4, lines 52 to 56; examples 11, 13<br>& DE 3231705 A1 & WO 84/00884 A1<br>& EP 116621 A1 | 1-2<br>3-16 |
| X<br>Y<br>A | JP 11-292746 A (3V Sigma S.P.A), 26 October, 1999 (26.10.99), Full text; particularly, Claims; Par. No. [0032]<br>& CA 2231080 A1 & US 5759525 A | 1-2<br>8-16<br>3-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 November, 2006 (17.11.06) | 12 December, 2006 (12.12.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/321925

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | JP 10-310514 A (3V Sigma S.P.A), 24 November, 1998 (24.11.98), Full text; particularly, Claims; Par. No. [0032] & EP 850935 A2 & CA 2231080 A | 1-2<br>8-16<br>3-7 |
| X<br>A | JP 09-176006 A (Masashi FUJII), 08 July, 1997 (08.07.97), Full text; particularly, Claims 1 to 3; column 3, line 11; table 1 (Family: none) | 1-2<br>3-16 |
| Y<br>A | Chemical Abstracts, 1989, vol.110, abstract no.160191 & Brunke, E. J. et al., Wetting and spreading as selection criteria of cosmetic oils, Seifen, Oele, Fette, Wachse, 1989, vol.115, no.2, pp.45-7 | 1-2,8-16<br>3-7 |
| A | WO 02/17861 A1 (L'OREAL), 07 March, 2002 (07.03.02), & CN 1394134 A & EP 1315472 A1 & FR 2813184 A1 & JP 2004-507483 T & US 2003/082125 A1 | 1-16 |
| A | JP 2001-158715 A (L'OREAL), 12 June, 2001 (12.06.01), & EP 1093844 A1 & FR 2799989 A1 | 1-16 |
| E,X | JP 2006-298834 A (Shiseido Co., Ltd.), 02 November, 2006 (02.11.06), Claims; examples (Family: none) | 1-7 |
| P,A | WO 2006/040349 A1 (Symrise Gmbh & Co. K.-G.), 20 April, 2006 (20.04.06), (Family: none) | 1-16 |

Form PCT/ISA/210 (continuation of second sheet) (April 2005)

**INTERNATIONAL SEARCH REPORT**

International application No.
PCT/JP2006/321925

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The matter common to the inventions according to claims 1-16 is a skin cosmetic comprising isononyl 2-ethylhexanoate and/or 2-ethylhexyl 2-ethylhexanoate. However, because the skin cosmetic is described in the following documents 1-3, etc., it is not novel, therefore, the matter is not a special technical feature within the meaning of PCT Rule 13.2, second sentence. Accordingly, the inventions according to claims 1-16 are not considered to be so linked as to form a single general inventive concept.

(continued to extra sheet)

1. ☐ As all required additional search fees were timely paid by the applicant, this international search report covers all searchable claims.
2. ☒ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2006/321925

Continuation of Box No.III of continuation of first sheet(2)

Document 1. US 5711939 A (Dragoco Gerberding & Co.), 27 January, 1998 (27.01.98)
2. JP 11-292746 A (3V Sigma S.P.A), 26 October, 1999 (26.10.99)
3. JP 09-176006 A (Masashi FUJII), 08 July, 1997 (08.07.97)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 47042502 B2
- JP 49000450 A
- JP 647941 A
- JP 2000169353 A


### Non-patent literature cited in the description


- Current Cosmetic Science. Yakuji Nippo Limited, 10 July 1992, 49